# EUROPEAN PATENT APPLICATION

(11) **EP 4 535 491 A1**
(43) Date of publication of application: **09.04.2025**
(21) Application number: 23835227.2
(22) Date of filing: 09.06.2023
(51) Int. Cl.: H01M 10/0567, C07D 233/34, C07D 239/10, C07D 239/52, C07D 243/04, C07D 249/12, C07D 251/08, C07D 251/10, C07D 251/22, C07F 1/02, H01M 10/052, H01M 10/054, H01M 10/0568, H01M 10/0569

(54) **NONAQUEOUS ELECTROLYTE SOLUTION, NONAQUEOUS ELECTROLYTE BATTERY, AND COMPOUND**

(30) Priority: 08.07.2022 JP 2022110786; 14.10.2022 JP 2022165369
(71) Applicant: Central Glass Company, Limited, Ube-shi, Yamaguchi 755-0001 (JP)
(72) Inventor: MIURA, Masahiro, Tokyo 101-0054 (JP); NAKAHARA, Keita, Tokyo 101-0054 (JP); IWASAKI, Susumu, Tokyo 101-0054 (JP); KATAOKA, Fuga, Tokyo 101-0054 (JP); MORINAKA, Takayoshi, Tokyo 101-0054 (JP)
(74) Representative: Manitz Finsterwald Patent- und Rechtsanwaltspartnerschaft mbB
(86) International application number: PCT/JP2023/021620
(87) International publication number: WO 2024/009691

(57) **Abstract**

According to a nonaqueous electrolyte solution containing: (I) at least one selected from the group consisting of a compound represented by Formula (1), a compound represented by Formula (2), a compound represented by Formula (3), a compound represented by Formula (4-1), a compound represented by Formula (4-2), a compound represented by Formula (4-3), a compound represented by Formula (5), and a compound represented by Formula (6) described in the specification; (II) a solute; and (III) a nonaqueous organic solvent, a nonaqueous electrolyte solution and a nonaqueous electrolyte solution battery having a low initial resistance value, and a compound that can be suitably used in the above nonaqueous electrolyte solution are provided.

## Description

### TECHNICAL FIELD

The present disclosure relates to a nonaqueous electrolyte solution, a nonaqueous electrolyte solution battery, and a compound.

### BACKGROUND ART

As measures for improving cycle characteristics, high-temperature storage characteristics, and durability of a nonaqueous electrolyte solution battery, optimization of various constituting elements of the battery including active materials of a positive electrode and a negative electrode has been hitherto studied. Techniques relating to a nonaqueous electrolyte solution have also been studied, and use of a variety of additives to prevent deterioration of a nonaqueous electrolyte solution at surfaces of active positive and negative electrodes due to decomposition of the nonaqueous electrolyte solution has been proposed.

For example, as a nonaqueous electrolyte solution which can constitute a lithium battery with excellent battery characteristics such as cycle characteristics, battery capacity, storage characteristics, and conductivity, Patent Literature 1 discloses a nonaqueous electrolyte solution for a lithium battery, in which the electrolyte is dissolved in a nonaqueous solvent, and the nonaqueous electrolyte solution further contains a pyrrolidone derivative.

Furthermore, Patent Literature 2 discloses that a nonaqueous electrolyte solution contains a nitrogen-containing heterocyclic compound such as pyrrolidone, oxazolidinone or imidazolidinone, so that a nonaqueous electrolyte secondary battery with excellent charge and discharge efficiency and capacity retention characteristics over a wide temperature range can be obtained.

### BACKGROUND ART LITERATURE

### PATENT LITERATURE

Patent Literature 1: JP2009-224281A
Patent Literature 2: JP5109213B

### SUMMARY OF INVENTION

### TECHNICAL PROBLEM

However, as a result of review, the inventors of the present invention have found that when five-membered ring compounds including an amide group are added, an increase in internal resistance is confirmed, and there is no or there is a small effect of improving initial input and output characteristics. Thus, there is room for further investigation of resistance characteristics, particularly of initial resistance characteristics.

The present disclosure is made in view of the above circumstances, and an object thereof is to provide a nonaqueous electrolyte solution and a nonaqueous electrolyte solution battery which can reduce an initial resistance value of a battery. Another object is to provide a compound which can be suitably used in the above nonaqueous electrolyte solution.

### SOLUTION TO PROBLEM

In view of such problems, the inventors of the present invention have conducted extensive research and discovered that a nonaqueous electrolyte solution battery having a low initial resistance value can be obtained by using a nonaqueous electrolyte solution containing: (I) at least one selected from the group consisting of a compound represented by Formula (1), a compound represented by Formula (2), a compound represented by Formula (3), a compound represented by Formula (4-1), a compound represented by Formula (4-2), a compound represented by Formula (4-3), a compound represented by Formula (5), and a compound represented by Formula (6) described below (hereinafter, also referred to as "component (I)"); (II) a solute (hereinafter, also referred to as "component (II)"); and (III) a nonaqueous organic solvent (hereinafter, also referred to as "component (III)"), thereby solving the above problems.

That is, the inventors of the present invention have found that the above problems can be solved by the following configurations.
[1] A nonaqueous electrolyte solution containing:
   (I) at least one selected from the group consisting of a compound represented by the following Formula (1), a compound represented by the following Formula (2), a compound represented by the following Formula (3), a compound represented by the following Formula (4-1), a compound represented by the following Formula (4-2), a compound represented by the following Formula (4-3), a compound represented by the following Formula (5), and a compound represented by the following Formula (6);
   (II) a solute; and
   (III) a nonaqueous organic solvent.

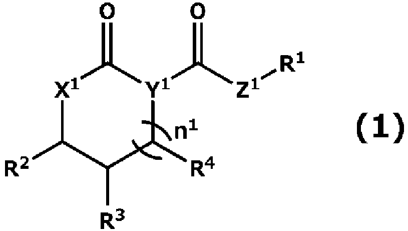

[In Formula (1), X¹ represents CH₂, NH, O, S, or SO₂
Y¹ represents CH or N.
Z¹ represents CH₂, O, or NR⁵.
R⁵ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R⁵ represents an alkali metal cation, a bond between a nitrogen atom and R⁵ represents an ionic bond.
R¹ represents PO(R_{f})₂ or SO₂R_{f}
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.
n¹ represents an integer of 1 to 3.
R², R³, and R⁴ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When there are a plurality of R⁴, the plurality of R⁴ may be the same or different.] 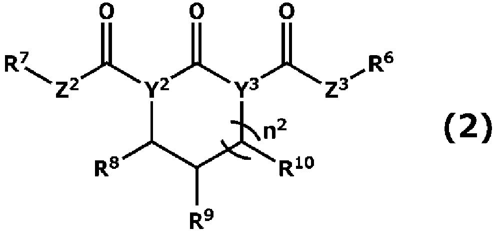
.

[In Formula (2), Y² and Y³ each independently represent CH or N.

Z² and Z³ each independently represent CH₂, O, or NR¹¹.

R¹¹ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R¹¹ represents an alkali metal cation, a bond between a nitrogen atom and R¹¹ represents an ionic bond.

R⁶ and R⁷ each independently represent PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

n² represents an integer of 1 to 3.

R⁸, R⁹, and R¹⁰ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When there are a plurality of R¹⁰, the plurality of R¹⁰ may be the same or different.]

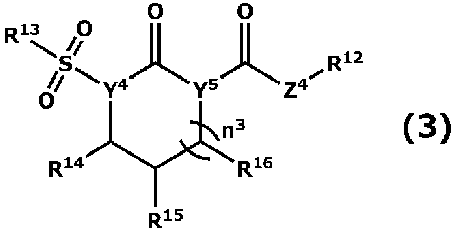

[In Formula (3), Y⁴ and Y⁵ each independently represent CH or N.

Z⁴ represents CH₂, O, or NR¹⁷.

R¹⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R¹⁷ represents an alkali metal cation, a bond between a nitrogen atom and R¹⁷ represents an ionic bond.

R¹² represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

R¹³ represents a halogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.

n³ represents an integer of 0 to 3.

R¹⁴, R¹⁵, and R¹⁶ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When there are a plurality of R¹⁶, the plurality of R¹⁶ may be the same or different. Note that when n³ represents 0, there is no R¹⁶, and a carbon atom to which R¹⁵ is bonded is directly bonded to Y⁵.]

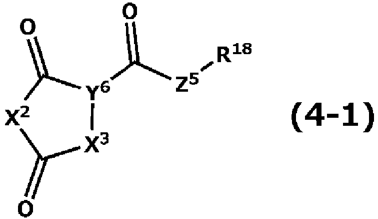

[In Formula (4-1), X² and X³ each independently represent CH₂, NH, O, or S.

Y⁶ represents CH or N.

Z⁵ represents CH₂, O, or NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R¹⁸ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.]

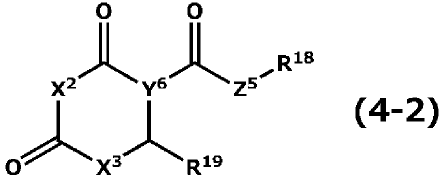

[In Formula (4-2), X² and X³ each independently represent CH₂, NH, O, or S.

Y⁶ represents CH or N.

Z⁵ represents CH₂, O, or NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R¹⁸ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

R¹⁹ represents a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.]

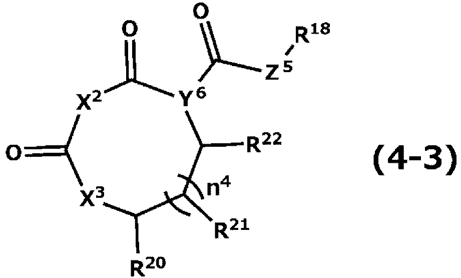

[In Formula (4-3), X² and X³ each independently represent CH₂, NH, O, or S.

Y⁶ represents CH or N.

Z⁵ represents CH₂, O, or NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R¹⁸ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

n⁴ represents 0 or 1.

R²⁰, R²¹, and R²² each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. Note that when n⁴ represents 0, there is no R²¹, and a carbon atom to which R²⁰ is bonded is directly bonded to a carbon atom to which R²² is bonded.]

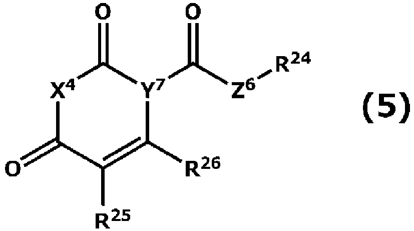

In Formula (5), X⁴ represents CH₂, NH, O, or S.

Y⁷ represents CH or N.

Z⁶ represents CH₂, O, or NR²⁷.

R²⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²⁷ represents an alkali metal cation, a bond between a nitrogen atom and R²⁷ represents an ionic bond.

R²⁴ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

R²⁵ and R²⁶ each independently represent a halogen atom, a hydrogen atom, a nitro group, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.]

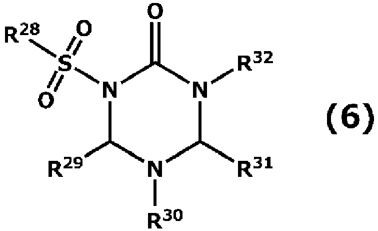

In Formula (6), R²⁸ represents a halogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.

R²⁹ and R³¹ each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.

R³⁰ and R³² each independently represent a hydrogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. A carbon-nitrogen unsaturated bond may be included in the alkyl group, the alkenyl group, or the alkynyl group, and any hydrogen atom of the alkyl group, the alkenyl group, or the alkynyl group may be substituted with a fluorine atom.]

[2]
The nonaqueous electrolyte solution according to [1], in which the above-described (I) is at least one selected from the group consisting of the compound represented by Formula (2), the compound represented by Formula (3), the compound represented by Formula (4-1), the compound represented by Formula (4-2), and the compound represented by Formula (5).

[3]
The nonaqueous electrolyte solution according to [1], in which R¹ in Formula (1) represents POF₂ or SO₂F.

[4]
The nonaqueous electrolyte solution according to [1] or [2], in which R⁶ and R⁷ in Formula (2) each independently represent POF₂ or SO₂F.

[5]
The nonaqueous electrolyte solution according to [1] or [2], in which R¹² in Formula (3) represents POF₂ or SO₂F.

[6]
The nonaqueous electrolyte solution according to [1] or [2], in which R¹⁸ in Formula (4-1), (4-2), or (4-3) represents POF₂ or SO₂F.

[7]
The nonaqueous electrolyte solution according to [1] or [2], in which R²⁴ in Formula (5) represents POF₂ or SO₂F.

[8]
The nonaqueous electrolyte solution according to [1], in which R²⁸ in Formula (6) represents a fluorine atom.

[9]
The nonaqueous electrolyte solution according to [1] or [3], in which R⁵ in Formula (1) represents a hydrogen atom, a lithium ion, a sodium ion, a linear alkyl group having 1 to 4 carbon atoms, or a branched alkyl group having 3 to 4 carbon atoms.

[10]
The nonaqueous electrolyte solution according to any one of [1], [2], and [4], in which R¹¹ in Formula (2) represents a hydrogen atom, a lithium ion, a sodium ion, a linear alkyl group having 1 to 4 carbon atoms, or a branched alkyl group having 3 to 4 carbon atoms.

[11]
The nonaqueous electrolyte solution according to any one of [1], [2], and [5], in which R¹⁷ in Formula (3) represents a hydrogen atom, a lithium ion, a sodium ion, a linear alkyl group having 1 to 4 carbon atoms, or a branched alkyl group having 3 to 4 carbon atoms.

[12]
The nonaqueous electrolyte solution according to any one of [1], [2], and [6], in which R²³ in Formula (4-1), (4-2), or (4-3) represents a hydrogen atom, a lithium ion, a sodium ion, a linear alkyl group having 1 to 4 carbon atoms, or a branched alkyl group having 3 to 4 carbon atoms.

[13]
The nonaqueous electrolyte solution according to any one of [1], [2], and [7], in which R²⁷ in Formula (5) represents a hydrogen atom, a lithium ion, a sodium ion, a linear alkyl group having 1 to 4 carbon atoms, or a branched alkyl group having 3 to 4 carbon atoms.

[14]
The nonaqueous electrolyte solution according to any one of [1] to [13], in which the solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

[15]
The nonaqueous electrolyte solution according to any one of [1] to [14], in which the nonaqueous organic solvent contains at least one selected from the group consisting of a cyclic ester, a chain ester, a cyclic ether, a chain ether, a sulfone compound, a sulfoxide compound, and an ionic liquid.

[16]
The nonaqueous electrolyte solution according to [15], in which the cyclic ester is a cyclic carbonate.

[17]
The nonaqueous electrolyte solution according to [16], in which the cyclic carbonate is at least one selected from the group consisting of ethylene carbonate, propylene carbonate, and fluoroethylene carbonate.

[18]
The nonaqueous electrolyte solution according to [15], in which the chain ester is a chain carbonate.

[19]
The nonaqueous electrolyte solution according to [18], in which the chain carbonate is at least one selected from the group consisting of ethyl methyl carbonate, dimethyl carbonate, diethyl carbonate, and methyl propyl carbonate.

[20]
The nonaqueous electrolyte solution according to any one of [1] to [19], in which a content of the above-described (I) is 0.01% to 10.0% by mass with respect to a total amount of the nonaqueous electrolyte solution.

[21]
The nonaqueous electrolyte solution according to any one of [1] to [20], further containing: at least one selected from the group consisting of vinylene carbonate, bis(oxalato)borate, difluorooxalatoborate, difluorobis(oxalato)phosphate, tetrafluorooxalatophosphate, tetrafluoro(malonato)phosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(picolinato)phosphate, 1,3-dimethyl-1,3-divinyl-1,3-di(1,1,1,3,3,3-hexafluoroisopropyl)disiloxane, t-butylbenzene, t-amylbenzene, fluorobenzene, and cyclohexylbenzene.

[22]
A nonaqueous electrolyte solution battery at least including:
a positive electrode;
a negative electrode;
a separator; and
the nonaqueous electrolyte solution according to any one of [1] to [21].

[23]
A compound represented by the following Formula (1).

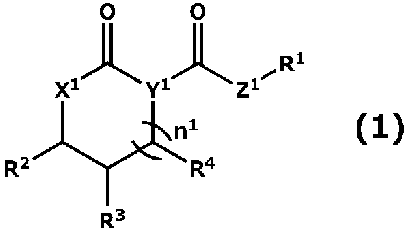

[In Formula (1), X¹ represents CH₂, NH, O, S, or SO₂.

Y¹ represents CH or N.

Z¹ represents CH₂, O, or NR⁵.

R⁵ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R⁵ represents an alkali metal cation, a bond between a nitrogen atom and R⁵ represents an ionic bond.

R¹ represents POF₂ or SO₂F.

n¹ represents an integer of 1 to 3, R², R³, and R⁴ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When there are a plurality of R⁴, the plurality of R⁴ may be the same or different.]

[24]
A compound represented by the following Formula (2).

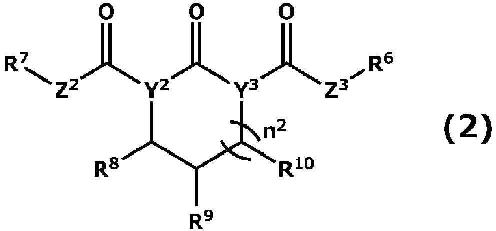

[In Formula (2), Y² and Y³ each independently represent CH or N.

Z² and Z³ each independently represent CH₂, O, or NR¹¹.

R¹¹ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R¹¹ represents an alkali metal cation, a bond between a nitrogen atom and R¹¹ represents an ionic bond.

R⁶ and R⁷ each independently represent POF₂ or SO₂F.

n² represents an integer of 1 to 3.

R⁸, R⁹, and R¹⁰ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When there are a plurality of R¹⁰, the plurality of R¹⁰ may be the same or different.]

[25]
A compound represented by the following Formula (3).

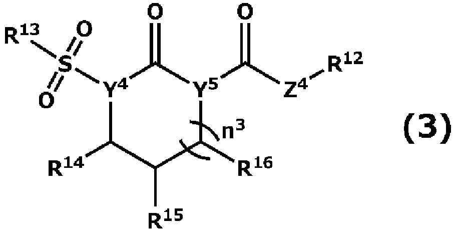

[In Formula (3), Y⁴ and Y⁵ each independently represent CH or N.

Z⁴ represents CH₂, O, or NR¹⁷.

R¹⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R¹⁷ represents an alkali metal cation, a bond between a nitrogen atom and R¹⁷ represents an ionic bond.

R¹² represents POF₂ or SO₂F.

R¹³ represents a halogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.

n³ represents an integer of 0 to 3.

R¹⁴, R¹⁵, and R¹⁶ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When there are a plurality of R¹⁶, the plurality of R¹⁶ may be the same or different. Note that when n³ represents 0, there is no R¹⁶, and a carbon atom to which R¹⁵ is bonded is directly bonded to Y⁵.]

[26]
A compound represented by the following Formula (4-1), the following Formula (4-2), or the following Formula (4-3).

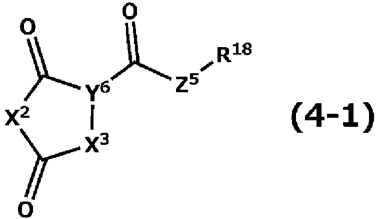

[In Formula (4-1), X² and X³ each independently represent CH₂, NH, O, or S.

Y⁶ represents CH or N.

Z⁵ represents CH₂, O, or NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R¹⁸ represents POF₂ or SO₂F.]

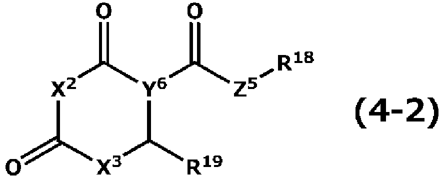

[In Formula (4-2), X² and X³ each independently represent CH₂, NH, O, or S.

Y⁶ represents CH or N.

Z⁵ represents CH₂, O, or NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R¹⁸ represents POF₂ or SO₂F.

R¹⁹ represents a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.]

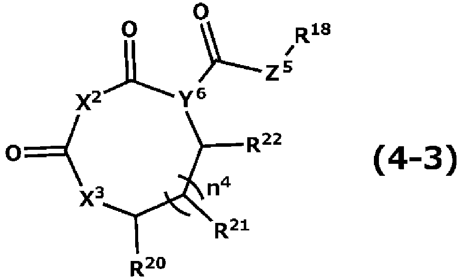

[In Formula (4-3), X² and X³ each independently represent CH₂, NH, O, or S.

Y⁶ represents CH or N.

Z⁵ represents CH₂, O, or NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R¹⁸ represents POF₂ or SO₂F.

n⁴ represents 0 or 1.

R²⁰, R²¹, and R²² each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. Note that when n⁴ represents 0, there is no R²¹, and a carbon atom to which R²⁰ is bonded is directly bonded to a carbon atom to which R²² is bonded.]

[27]
A compound represented by the following Formula (5).

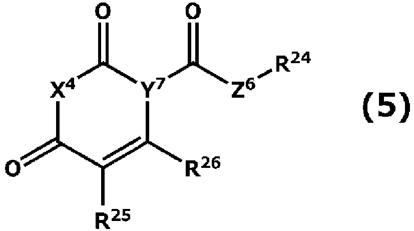

[In Formula (5), X⁴ represents CH₂, NH, or O.

Y⁷ represents CH or N.

Z⁶ represents CH₂, O, or R²⁷.

R²⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²⁷ represents an alkali metal cation, a bond between a nitrogen atom and R²⁷ represents an ionic bond.

R²⁴ represents POF₂ or SO₂F.

R²⁵ and R²⁶ each independently represent a halogen atom, a hydrogen atom, a nitro group, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.]

[28]
A compound represented by the following Formula (6).

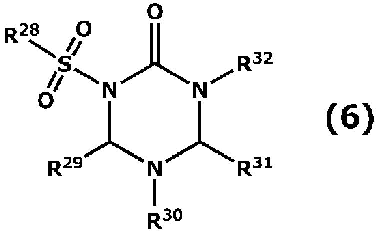

[In Formula (6), R²⁸ represents a fluorine atom.

R²⁹ and R³¹ each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom.

R³⁰ and R³² each independently represent a hydrogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. A carbon-nitrogen unsaturated bond may be included in the alkyl group, the alkenyl group, or the alkynyl group, and any hydrogen atom of the alkyl group, the alkenyl group, or the alkynyl group may be substituted with a fluorine atom.]

### EFFECTS OF INVENTION

According to the present disclosure, a nonaqueous electrolyte solution and a nonaqueous electrolyte solution battery which can reduce an initial resistance value can be provided. The present disclosure also allows for providing a compound that can be suitably used in the above nonaqueous electrolyte solution.

### DESCRIPTION OF EMBODIMENTS

Configurations and combinations thereof in the following embodiments are merely examples, and additions, replacements, and other changes of the configurations may be made without departing from the scope of the present disclosure. In addition, the present disclosure is not limited by the embodiments, but only limited by the scope of claims.

In the present description, the expression "to" means to include numerical values before and after "to" as a lower limit value and an upper limit value, respectively.

An initial resistance value in the present specification means a resistance value of a nonaqueous electrolyte solution battery immediately after an initial charge and discharge operation for battery stabilization. Specifically, the initial resistance value refers to a resistance value obtained by initial impedance measurement after three cycles of a charge and discharge operation for battery stabilization.

### [1. Nonaqueous Electrolyte Solution]

A nonaqueous electrolyte solution according to the present disclosure is a nonaqueous electrolyte solution containing: (I) at least one selected from the group consisting of a compound represented by Formula (1), a compound represented by Formula (2), a compound represented by Formula (3), a compound represented by Formula (4-1), a compound represented by Formula (4-2), a compound represented by Formula (4-3), a compound represented by Formula (5), and a compound represented by Formula (6); (II) a solute; and (III) a nonaqueous organic solvent.

### <Component (I)>

The nonaqueous electrolyte solution according to the present disclosure contains a component (I), that is, at least one selected from the group consisting of the compound represented by Formula (1), the compound represented by Formula (2), the compound represented by Formula (3), the compound represented by Formula (4-1), the compound represented by Formula (4-2), the compound represented by Formula (4-3), the compound represented by Formula (5), and the compound represented by Formula (6).

When the nonaqueous electrolyte solution containing the above component (I) is used in a nonaqueous electrolyte solution battery (for example, a lithium ion secondary battery or a sodium ion secondary battery), the component (I) decomposes on at least one of a positive electrode and a negative electrode, and forms a film having good cation conductivity on a surface of at least one of the positive electrode and the negative electrode. It is considered that this film prevents direct contact between a nonaqueous organic solvent or solute and an electrode active material, and reduces cation dissociation energy of the solute. The inventors of the present invention assume that an effect of reducing initial resistance of the nonaqueous electrolyte solution battery is exhibited as a result of the above.

The above component (I) may be at least one selected from the group consisting of the compound represented by Formula (2), the compound represented by Formula (3), the compound represented by Formula (4-1), the compound represented by Formula (4-2), and the compound represented by Formula (5).

The compound represented by Formula (1) is described below.

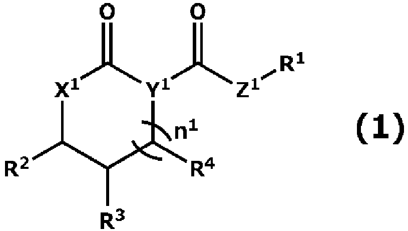

X¹ represents CH₂, NH, O, S, or SO₂. Y¹ represents CH or N. Z¹ represents CH₂, O, or NR⁵.

X¹ may be CH₂ or O.

Y¹ may be N.

Z¹ may be NR⁵.

R⁵ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R⁵ represents an alkali metal cation, a bond between a nitrogen atom and R⁵ represents an ionic bond.

R⁵ may be a hydrogen atom, an alkali metal cation, a linear alkyl group having 1 to 4 carbon atoms, or a branched alkyl group having 3 to 4 carbon atoms, may be a hydrogen atom, a lithium ion, a sodium ion, a linear alkyl group having 1 to 4 carbon atoms, or a branched alkyl group having 3 to 4 carbon atoms, or may be a hydrogen atom, a lithium ion, a sodium ion, or a methyl group.

R¹ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

Specific examples of R_{f} representing a linear alkyl group having 1 to 12 carbon atoms or a branched alkyl group having 3 to 12 carbon atoms include a methyl group, an ethyl group, an n-propyl group, an isopropyl group, an n-butyl group, a sec-butyl group, an isobutyl group, a tert-butyl group, and an n-pentyl group.

Specific examples of R_{f} representing a linear alkenyl group having 2 to 12 carbon atoms or a branched alkenyl group having 3 to 12 carbon atoms include a vinyl group, an allyl group, a 1-propenyl group, an isopropenyl group, a 2-butenyl group, and a 1,3-butadienyl group.

Specific examples of R_{f} representing a linear alkynyl group having 2 to 12 carbon atoms or a branched alkynyl group having 4 to 12 carbon atoms include an ethynyl group, a 2-propynyl group, and a 1,1-dimethyl-2-propynyl group.

At least one of an oxygen atom and a nitrogen atom may be included between a carbon atom-carbon atom bond in the alkyl group, the alkenyl group, or the alkynyl group. Examples include a 2-methoxyethyl group, a 2-ethoxyethyl group, an (allyloxy)methyl group, a (propargyloxy)methyl group, a (dimethylamino)ethyl group, and a (diethylamino)ethyl group.

Any hydrogen atom of the alkyl group, the alkenyl group, or the alkynyl group may be substituted with a fluorine atom. Examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a 1,1-bis(trifluoromethyl)-2-propenyl group, and a 3,3,3-trifluoropropynyl group.

R¹ may be POF₂ or SO₂F, or may be SO₂F.

R² to R⁴ each independently represent a hydrogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R² to R⁴ may each independently represent a hydrogen atom or an alkyl group having 1 to 4 carbon atoms, may be a hydrogen atom or a methyl group, or may be a hydrogen atom.

n¹ represents an integer of 1 to 3, n¹ may represent 1 or 2, or n¹ may represent 1.

Specifically, the compound represented by Formula (1) may be at least one selected from the group consisting of compounds represented by the following Formulas (1-1-a) to (1-1-z), (1-2-a) to (1-2-z), and (1-3-a) to (1-3-z).

At least one selected from the group consisting of the compound represented by Formula (1-1-a) (also referred to as a compound (1-1-a)), the compound represented by Formula (1-1-g) (also referred to as a compound (1-1-g)), the compound represented by Formula (1-1-q) (also referred to as a compound (1-1-q)), the compound represented by Formula (1-1-x) (also referred to as a compound (1-1-x)), the compound represented by Formula (1-2-g) (also referred to as a compound (1-2-g)), the compound represented by Formula (1-2-q) (also referred to as a compound (1-2-q)), the compound represented by Formula (1-2-x) (also referred to as a compound (1-2-x)), the compound represented by Formula (1-3-a) (also referred to as a compound (1-3-a)), the compound represented by Formula (1-3-g) (also referred to as a compound (1-3-g)), and the compound represented by Formula (1-3-x) (also referred to as a compound (1-3-x)) is more preferred.

At least one selected from the group consisting of the compound (1-1-a), the compound (1-1-g), the compound (1-1-q), the compound (1-1-x), and the compound (1-2-g) is furthermore preferred.

The compound (1-1-g) is particularly preferred.

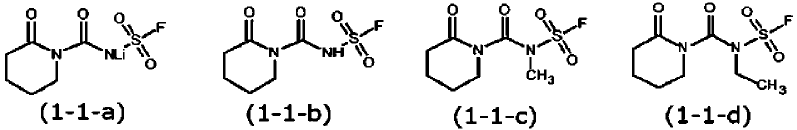

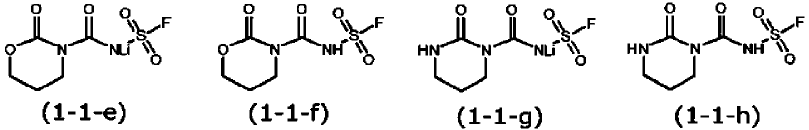

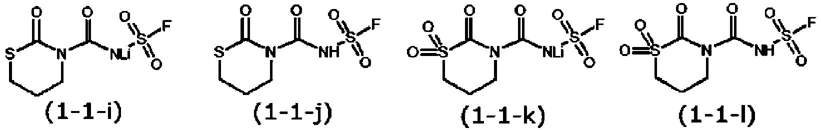

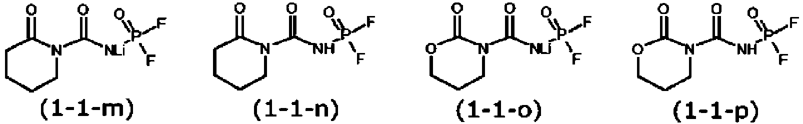

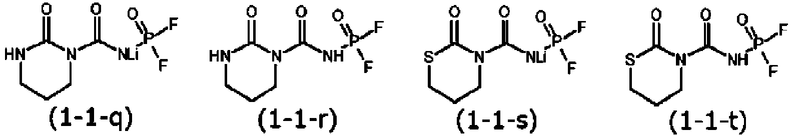

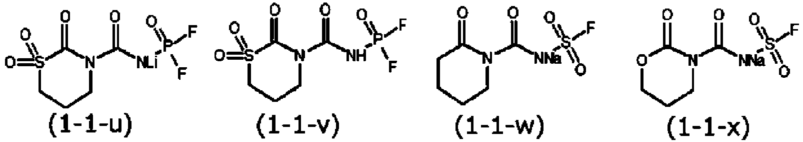

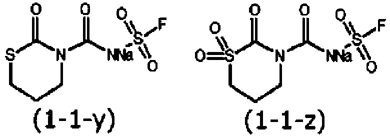

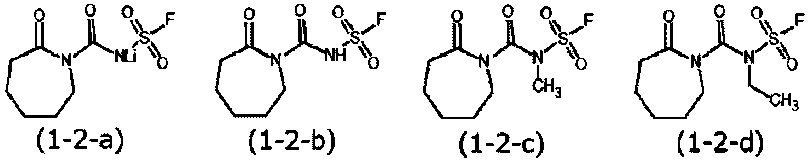

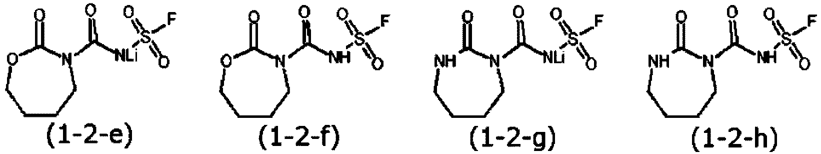

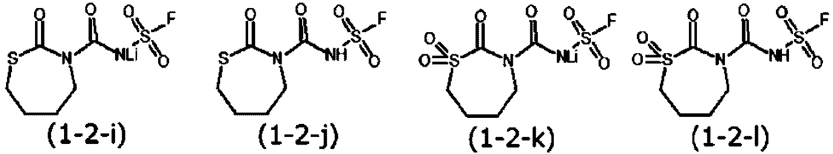

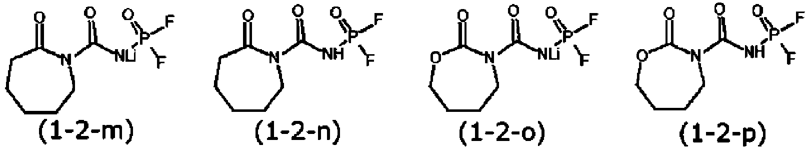

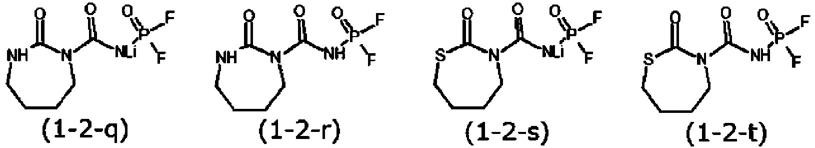

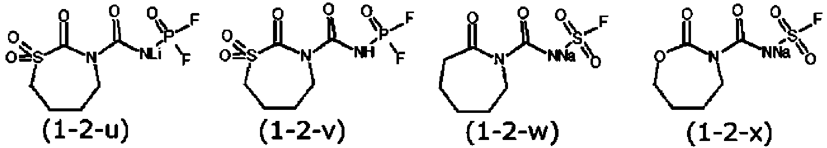

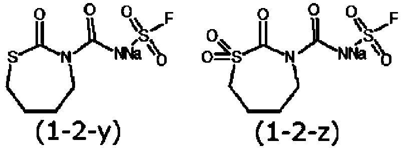

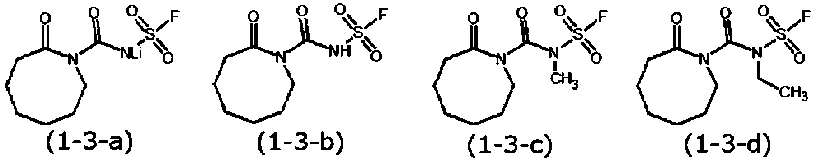

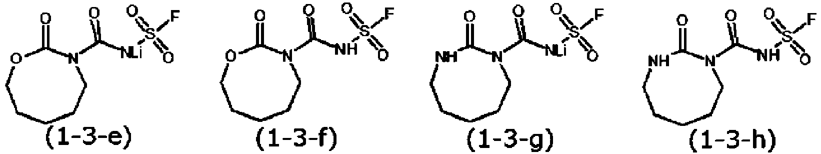

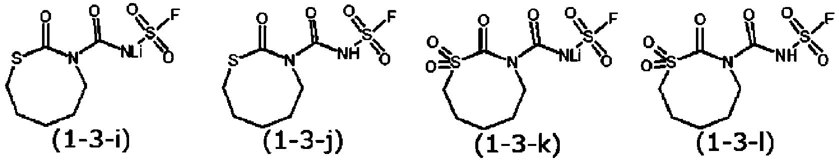

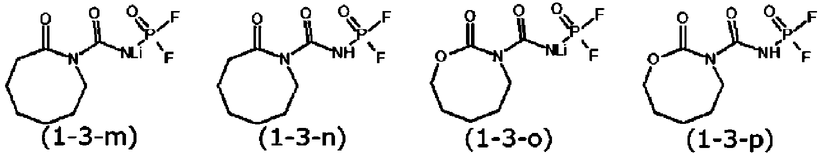

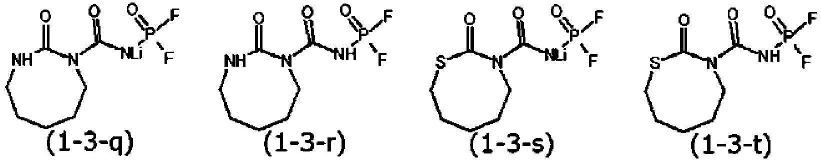

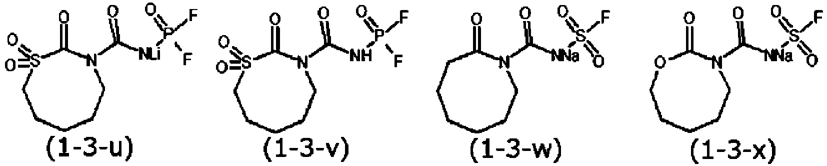

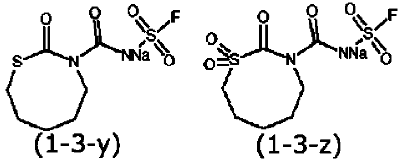

The compound represented by Formula (2) is described below.

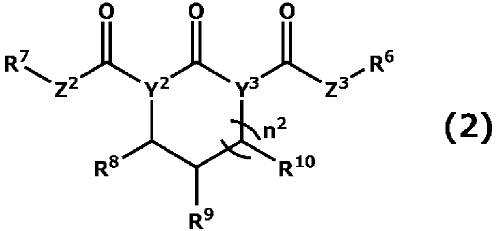

Y² and Y³ each independently represent CH or N. Z² and Z³ each independently represent CH₂, O, or NR¹¹.

Both of Y² and Y³ may be N.

Both of Z² and Z³ may be NR¹¹.

R¹¹ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R¹¹ represents an alkali metal cation, a bond between a nitrogen atom and R¹¹ represents an ionic bond.

R¹¹ may be a hydrogen atom, an alkali metal cation, a linear alkyl group having 1 to 4 carbon atoms, or a branched alkyl group having 3 to 4 carbon atoms, may be a hydrogen atom, a lithium ion, a sodium ion, a linear alkyl group having 1 to 4 carbon atoms, or a branched alkyl group having 3 to 4 carbon atoms, or may be a hydrogen atom, a lithium ion, a sodium ion, or a methyl group.

R⁶ and R⁷ each independently represent PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

Specific examples of R_{f} representing a linear alkyl group having 1 to 12 carbon atoms or a branched alkyl group having 3 to 12 carbon atoms include a methyl group, an ethyl group, an n-propyl group, an isopropyl group, an n-butyl group, a sec-butyl group, an isobutyl group, a tert-butyl group, and an n-pentyl group.

Specific examples of R_{f} representing a linear alkenyl group having 2 to 12 carbon atoms or a branched alkenyl group having 3 to 12 carbon atoms include a vinyl group, an allyl group, a 1-propenyl group, an isopropenyl group, a 2-butenyl group, and a 1,3-butadienyl group.

Specific examples of R_{f} representing a linear alkynyl group having 2 to 12 carbon atoms or a branched alkynyl group having 4 to 12 carbon atoms include an ethynyl group, a 2-propynyl group, and a 1,1-dimethyl-2-propynyl group.

At least one of an oxygen atom and a nitrogen atom may be included between a carbon atom-carbon atom bond in the alkyl group, the alkenyl group, or the alkynyl group. Examples include a 2-methoxyethyl group, a 2-ethoxyethyl group, an (allyloxy)methyl group, a (propargyloxy)methyl group, a (dimethylamino)ethyl group, and a (diethylamino)ethyl group.

Any hydrogen atom of the alkyl group, the alkenyl group, or the alkynyl group may be substituted with a fluorine atom. Examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a 1,1-bis(trifluoromethyl)-2-propenyl group, and a 3,3,3-trifluoropropynyl group.

R⁶ and R⁷ may each independently represent POF₂ or SO₂F, or may be SO₂F.

R⁸ to R¹⁰ each independently represent a hydrogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R⁸ to R¹⁰ may each independently represent a hydrogen atom or an alkyl group having 1 to 4 carbon atoms, may be a hydrogen atom or a methyl group, or may be a hydrogen atom.

n² represents an integer of 1 to 3, n² may represent 1 or 2, or n² may represent 1.

Specifically, the compound represented by Formula (2) may be at least one selected from the group consisting of compounds represented by the following Formulas (2-1-a) to (2-1-r), (2-2-a) to (2-2-r), and (2-3-a) to (2-3-r).

At least one selected from the group consisting of the compound represented by Formula (2-1-a) (also referred to as a compound (2-1-a)), the compound represented by Formula (2-1-d) (also referred to as a compound (2-1-d)), the compound represented by Formula (2-1-i) (also referred to as a compound ((2-1-i)), the compound represented by Formula (2-1-k) (also referred to as a compound (2-1-k)), the compound represented by Formula (2-1-1) (also referred to as a compound (2-1-l)), and the compound represented by Formula (2-2-a) (also referred to as a compound (2-2-a)) is more preferred.

At least one selected from the group consisting of the compound (2-1-a), the compound (2-1-d), the compound (2-1-1), and the compound (2-2-a) is furthermore preferred.

At least one selected from the group consisting of the compound (2-1-a), the compound (2-1-d), and the compound (2-1-i) is particularly preferred.

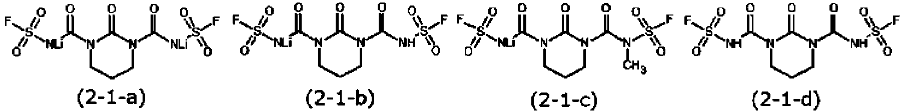

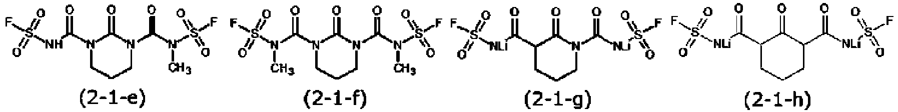

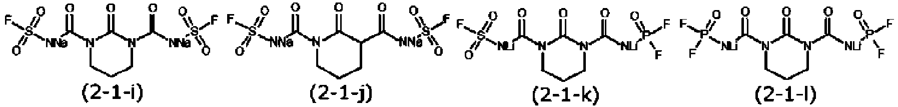

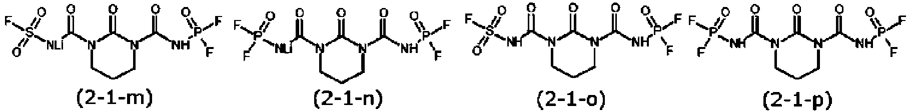

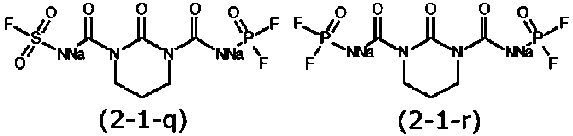

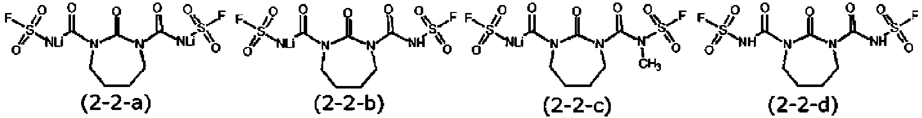

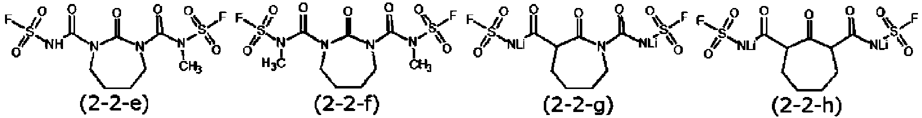

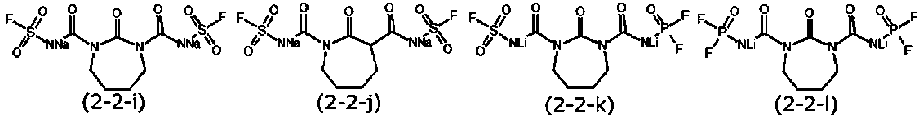

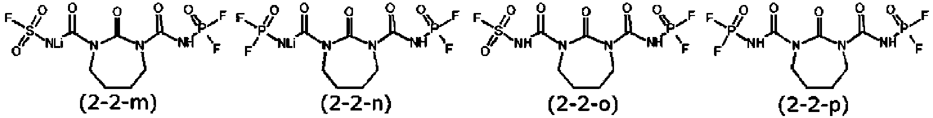

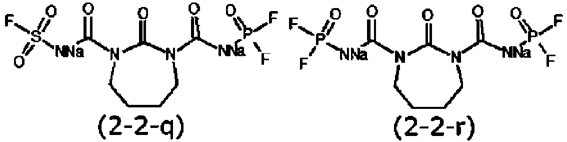

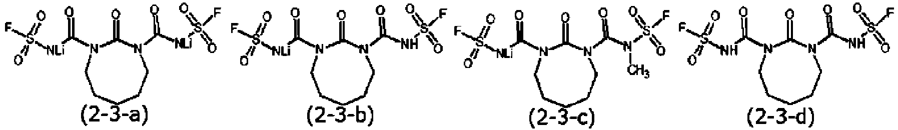

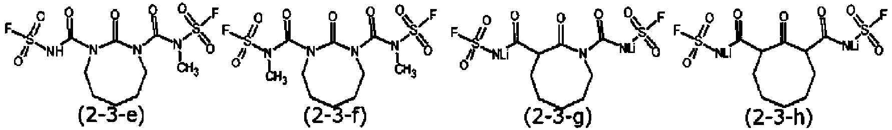

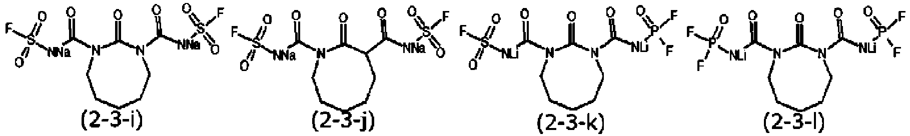

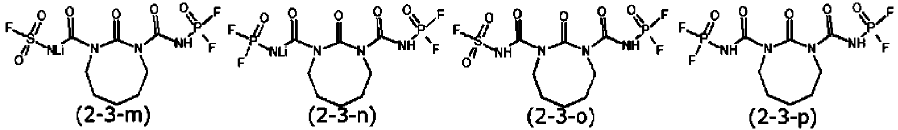

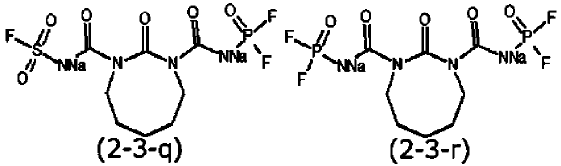

The compound represented by Formula (3) is described below.

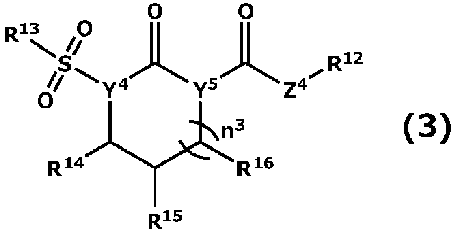

Y⁴ and Y⁵ each independently represent CH or N. Z⁴ represents CH₂, O, or NR¹⁷.

Both of Y⁴ and Y⁵ may be N.

Z⁴ may be NR¹⁷.

R¹⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R¹⁷ represents an alkali metal cation, a bond between a nitrogen atom and R¹⁷ represents an ionic bond.

R¹⁷ may be a hydrogen atom, an alkali metal cation, a linear alkyl group having 1 to 4 carbon atoms, or a branched alkyl group having 3 to 4 carbon atoms, may be a hydrogen atom, a lithium ion, a sodium ion, a linear alkyl group having 1 to 4 carbon atoms, or a branched alkyl group having 3 to 4 carbon atoms, or may be a hydrogen atom, a lithium ion, a sodium ion, or a methyl group.

R¹² represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

Specific examples of R_{f} representing a linear alkyl group having 1 to 12 carbon atoms or a branched alkyl group having 3 to 12 carbon atoms include a methyl group, an ethyl group, an n-propyl group, an isopropyl group, an n-butyl group, a sec-butyl group, an isobutyl group, a tert-butyl group, and an n-pentyl group.

Specific examples of R_{f} representing a linear alkenyl group having 2 to 12 carbon atoms or a branched alkenyl group having 3 to 12 carbon atoms include a vinyl group, an allyl group, a 1-propenyl group, an isopropenyl group, a 2-butenyl group, and a 1,3-butadienyl group.

Specific examples of R_{f} representing a linear alkynyl group having 2 to 12 carbon atoms or a branched alkynyl group having 4 to 12 carbon atoms include an ethynyl group, a 2-propynyl group, and a 1,1-dimethyl-2-propynyl group.

At least one of an oxygen atom and a nitrogen atom may be included between a carbon atom-carbon atom bond in the alkyl group, the alkenyl group, or the alkynyl group. Examples include a 2-methoxyethyl group, a 2-ethoxyethyl group, an (allyloxy)methyl group, a (propargyloxy)methyl group, a (dimethylamino)ethyl group, and a (diethylamino)ethyl group.

Any hydrogen atom of the alkyl group, the alkenyl group, or the alkynyl group may be substituted with a fluorine atom. Examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a 1,1-bis(trifluoromethyl)-2-propenyl group, and a 3,3,3-trifluoropropynyl group.

R¹² may be POF₂ or SO₂F, or may be SO₂F.

R¹³ represents a halogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R¹³ may be a fluorine atom or an alkyl group having 1 to 4 carbon atoms, or may be a fluorine atom or a methyl group.

R¹⁴ to R¹⁶ each independently represent a hydrogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R¹⁴ to R¹⁶ may each independently represent a hydrogen atom or an alkyl group having 1 to 4 carbon atoms, may be a hydrogen atom or a methyl group, or may be a hydrogen atom.

n³ represents an integer of 0 to 3, n³ may represent an integer of 0 to 2, or n³ may represent 0 or 1. Note that when n³ represents 0, there is no R¹⁶, and a carbon atom to which R¹⁵ is bonded is directly bonded to Y⁵.

Specifically, the compound represented by Formula (3) may be at least one selected from the group consisting of compounds represented by the following Formulas (3-1-a) to (3-1-z), (3-2-a) to (3-2-z), (3-3-a) to (3-3-z), and (3-4-a) to (3-4-z).

At least one selected from the group consisting of the compound represented by Formula (3-1-a) (also referred to as a compound (3-1-a)), the compound represented by Formula (3-1-e) (also referred to as a compound (3-1-e)), the compound represented by Formula (3-1-q) (also referred to as a compound ((3-1-q)), the compound represented by Formula (3-1-y) (also referred to as a compound (3-1-y)), the compound represented by Formula (3-2-e) (also referred to as a compound (3-2-e)), the compound represented by Formula (3-2-q) (also referred to as a compound (3-2-q)), and the compound represented by Formula (3-2-y) (also referred to as a compound (3-2-y)) is more preferred.

At least one selected from the group consisting of the compound (3-1-a), the compound (3-1-e), the compound (3-1-y), the compound (3-2-e), and the compound (3-2-y) is furthermore preferred.

At least one selected from the group consisting of the compound (3-1-a), the compound (3-1-e), and the compound (3-2-e) is particularly preferred.

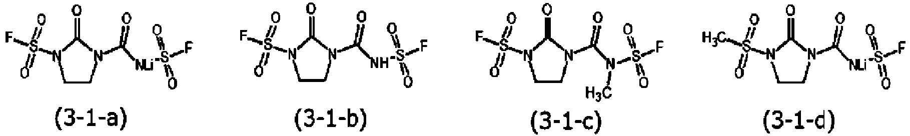

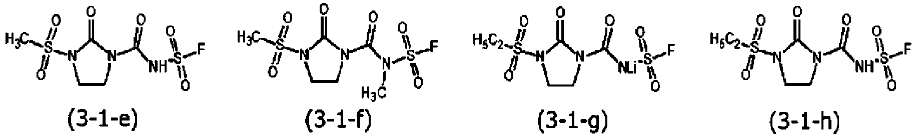

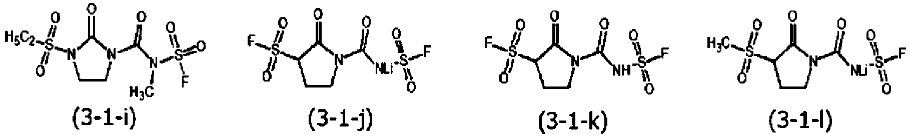

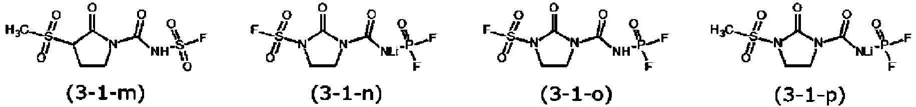

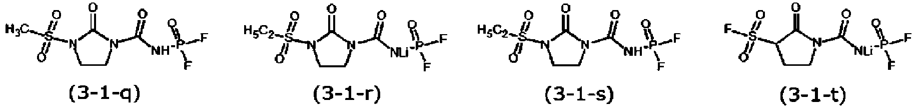

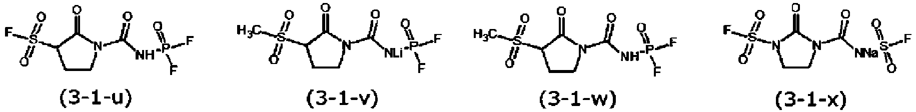

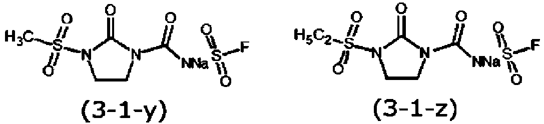

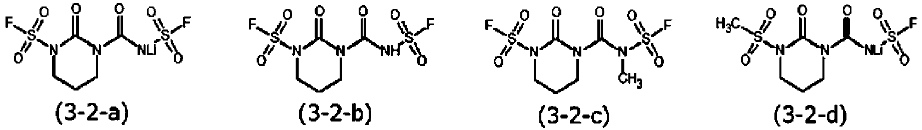

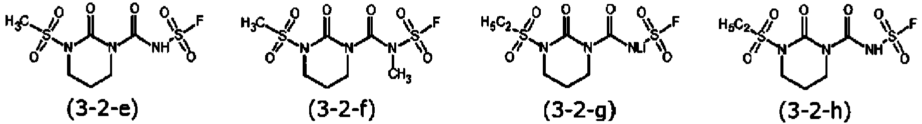

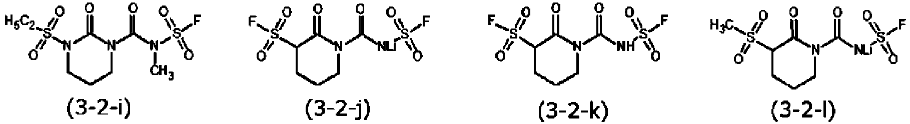

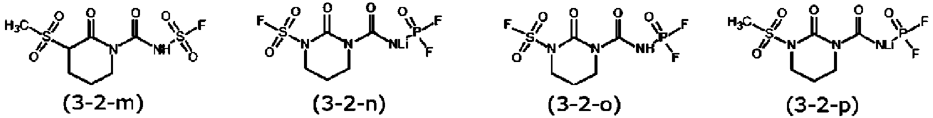

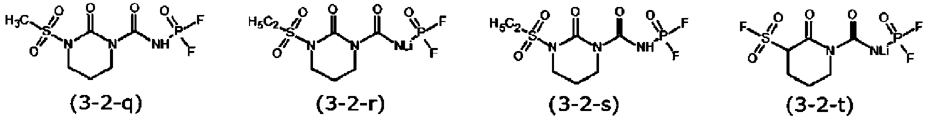

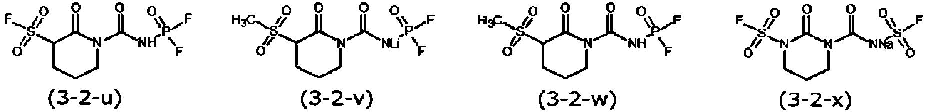

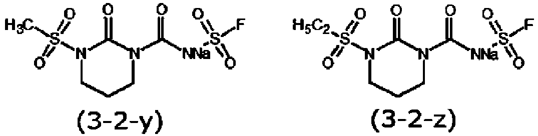

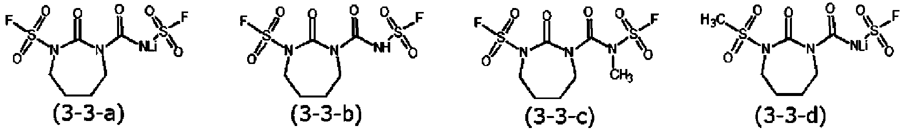

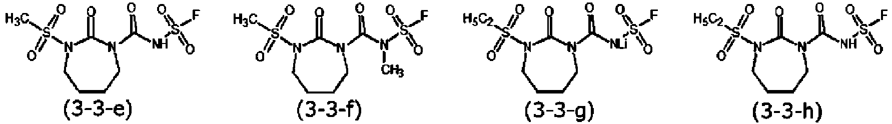

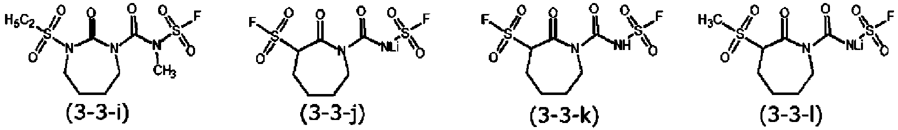

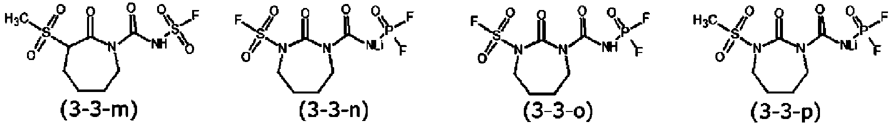

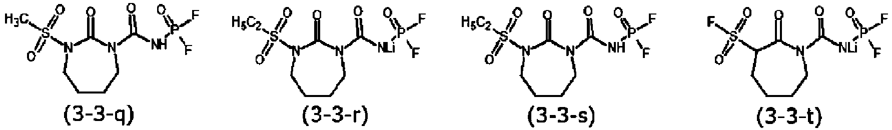

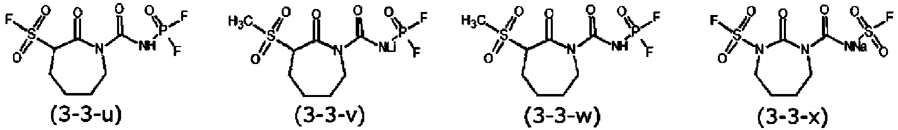

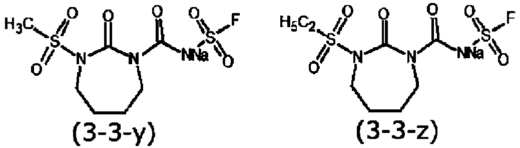

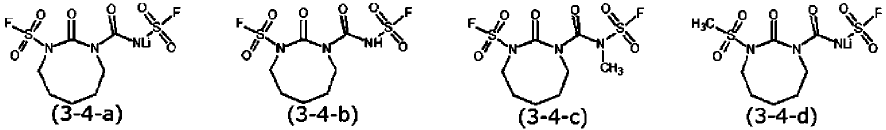

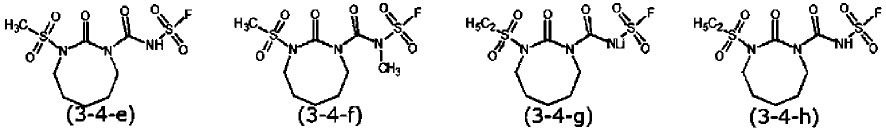

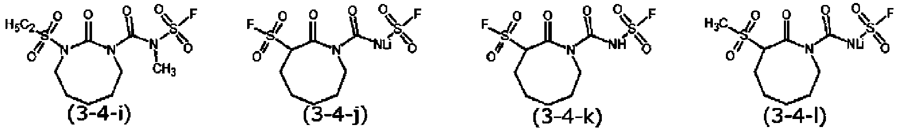

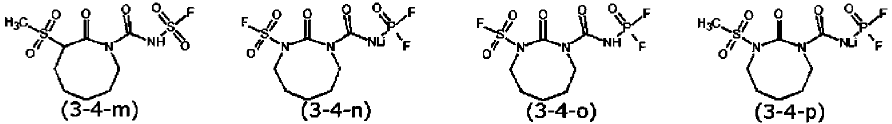

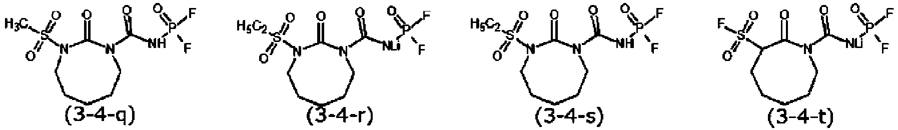

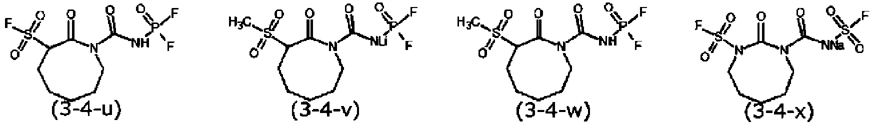

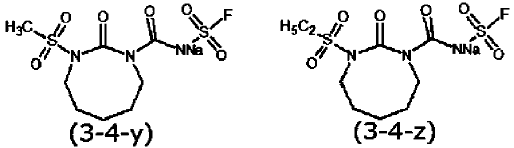

A compound represented by Formula (4-1), a compound represented by Formula (4-2), and a compound represented by Formula (4-3) are described below.

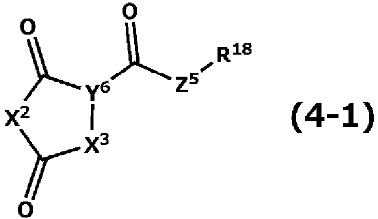

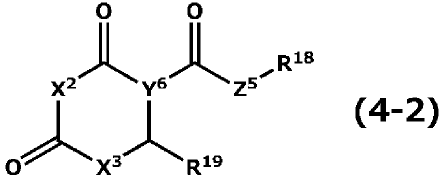

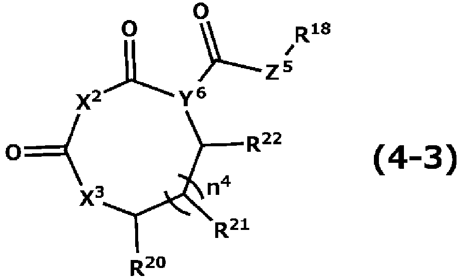

X² and X³ each independently represent CH₂, NH, O, or S. Y⁶ represents CH or N. Z⁵ represents CH₂, O, or NR²³.

X² may be NH or O.

X³ may be CH₂ or NH.

Y⁶ may be N.

Z⁵ may be NR²³.

R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²³ represents an alkali metal cation, a bond between a nitrogen atom and R²³ represents an ionic bond.

R²³ may be a hydrogen atom, an alkali metal cation, a linear alkyl group having 1 to 4 carbon atoms, or a branched alkyl group having 3 to 4 carbon atoms, may be a hydrogen atom, a lithium ion, a sodium ion, a linear alkyl group having 1 to 4 carbon atoms, or a branched alkyl group having 3 to 4 carbon atoms, or may be a hydrogen atom, a lithium ion, a sodium ion, or a methyl group.

R¹⁸ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

Specific examples of R_{f} representing a linear alkyl group having 1 to 12 carbon atoms or a branched alkyl group having 3 to 12 carbon atoms include a methyl group, an ethyl group, an n-propyl group, an isopropyl group, an n-butyl group, a sec-butyl group, an isobutyl group, a tert-butyl group, and an n-pentyl group.

Specific examples of R_{f} representing a linear alkenyl group having 2 to 12 carbon atoms or a branched alkenyl group having 3 to 12 carbon atoms include a vinyl group, an allyl group, a 1-propenyl group, an isopropenyl group, a 2-butenyl group, and a 1,3-butadienyl group.

Specific examples of R_{f} representing a linear alkynyl group having 2 to 12 carbon atoms or a branched alkynyl group having 4 to 12 carbon atoms include an ethynyl group, a 2-propynyl group, and a 1,1-dimethyl-2-propynyl group.

At least one of an oxygen atom and a nitrogen atom may be included between a carbon atom-carbon atom bond in the alkyl group, the alkenyl group, or the alkynyl group. Examples include a 2-methoxyethyl group, a 2-ethoxyethyl group, an (allyloxy)methyl group, a (propargyloxy)methyl group, a (dimethylamino)ethyl group, and a (diethylamino)ethyl group.

Any hydrogen atom of the alkyl group, the alkenyl group, or the alkynyl group may be substituted with a fluorine atom. Examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a 1,1-bis(trifluoromethyl)-2-propenyl group, and a 3,3,3-trifluoropropynyl group.

R¹⁸ may be POF₂ or SO₂F, or may be SO₂F.

R¹⁹, R²⁰, R²¹, and R²² each represent a halogen atom, a hydrogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R¹⁹, R²⁰, R²¹, and R²² may be a hydrogen atom or an alkyl group having 1 to 4 carbon atoms, may be a hydrogen atom or a methyl group, or may be a hydrogen atom.

n⁴ is 0 or 1. Note that when n⁴ represents 0, there is no R²¹, and a carbon atom to which R²⁰ is bonded is directly bonded to a carbon atom to which R²² is bonded.

Specifically, the compound represented by Formula (4-1), Formula (4-2), or Formula (4-3) may be at least one selected from the group consisting of compounds represented by the following Formulas (4-1-a) to (4-1-d'), (4-2-a) to (4-2-d'), (4-3-a) to (4-3-d'), and (4-4-a) to (4-4-d').

At least one selected from the group consisting of the compound represented by Formula (4-1-g) (also referred to as a compound (4-1-g)), the compound represented by Formula (4-1-j) (also referred to as a compound (4-1-j)), the compound represented by Formula (4-1-u) (also referred to as a compound ((4-1-u)), the compound represented by Formula (4-1-a') (also referred to as a compound (4-1-a')), the compound represented by Formula (4-1-b') (also referred to as a compound (4-1-b')), the compound represented by Formula (4-2-j) (also referred to as a compound (4-2-j)), and the compound represented by Formula (4-2-c') (also referred to as a compound (4-2-c')) is more preferred.

At least one selected from the group consisting of the compound (4-1-g), the compound (4-1 -j), the compound (4-1-u), and the compound (4-2-j) is furthermore preferred.

At least one selected from the group consisting of the compound (4-1-j) and the compound (4-2-j) is particularly preferred.

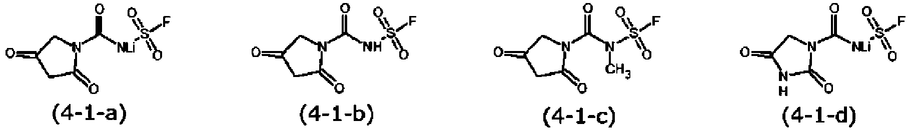

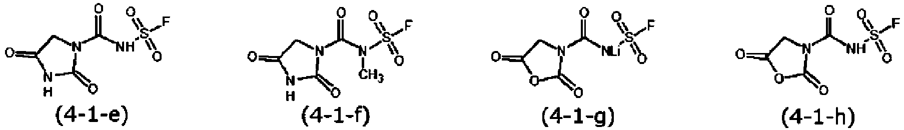

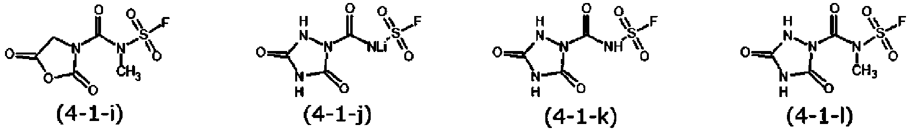

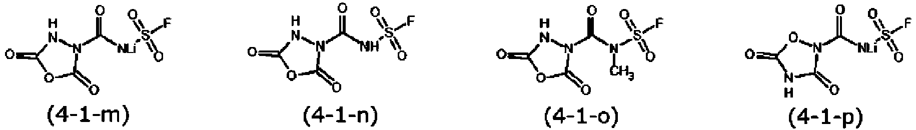

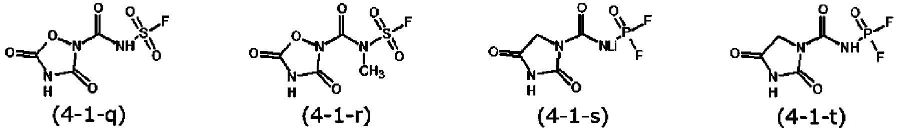

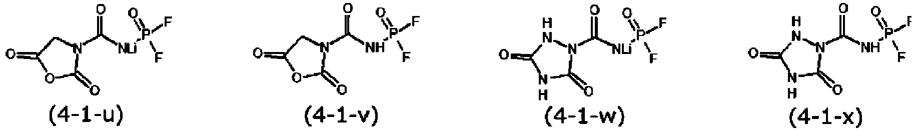

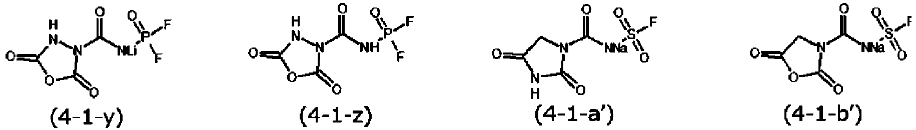

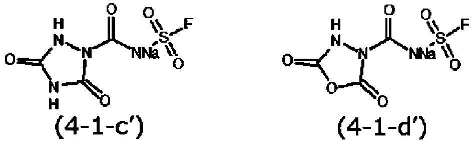

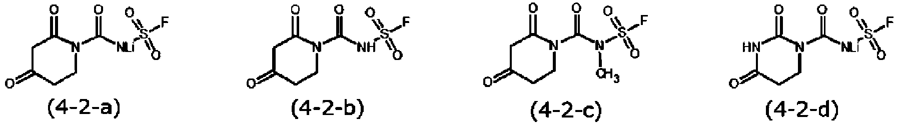

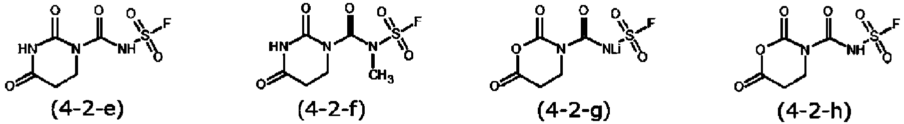

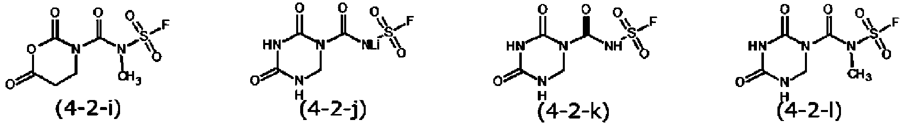

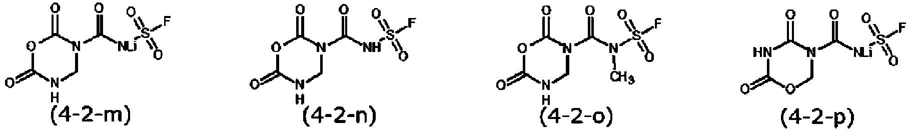

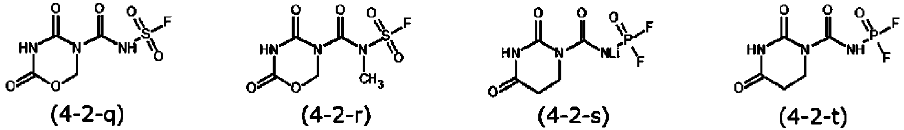

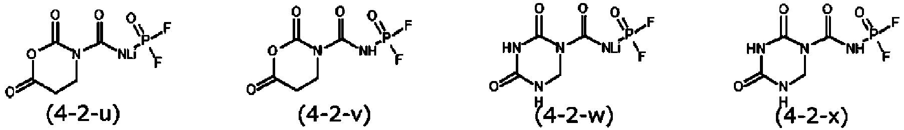

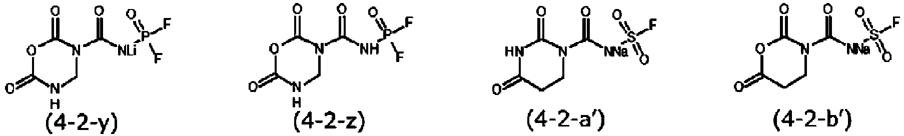

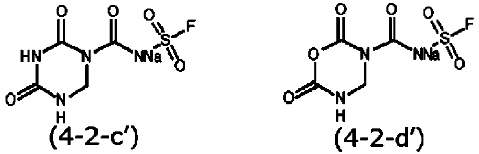

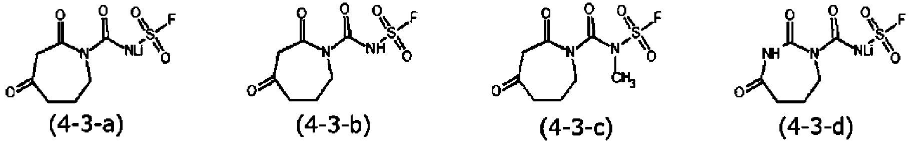

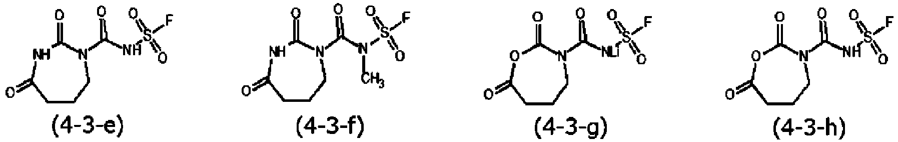

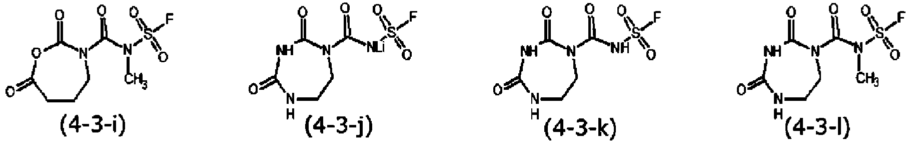

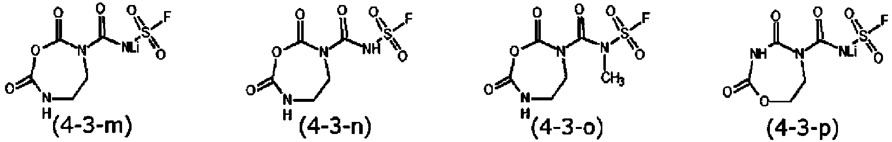

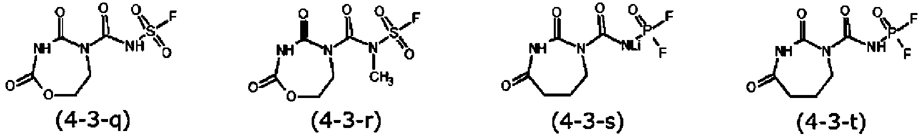

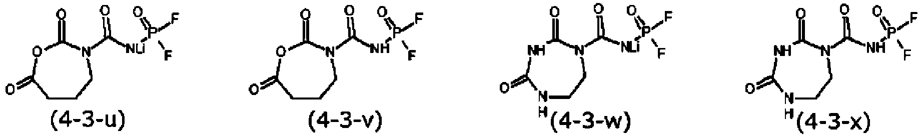

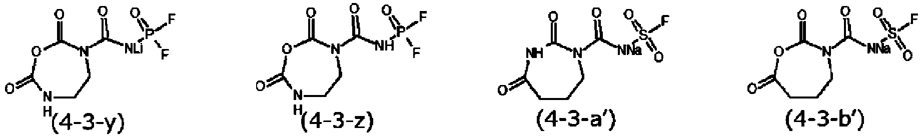

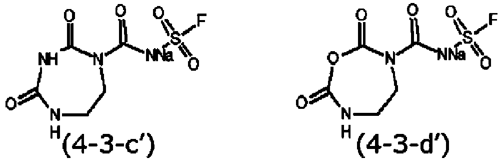

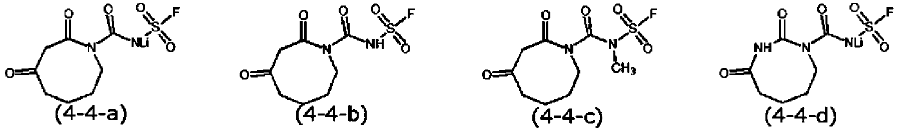

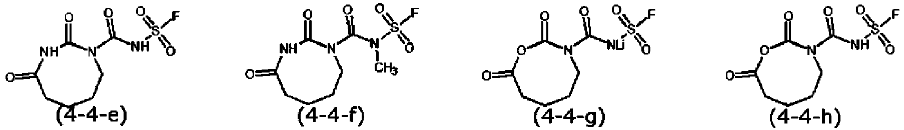

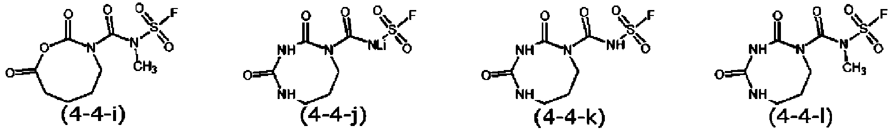

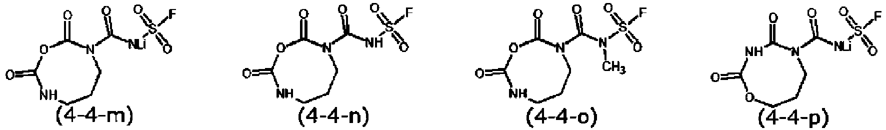

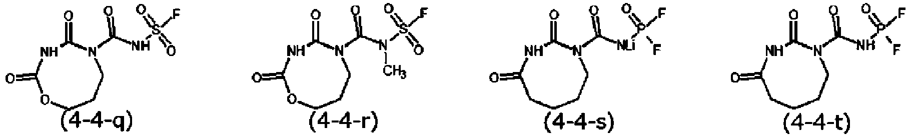

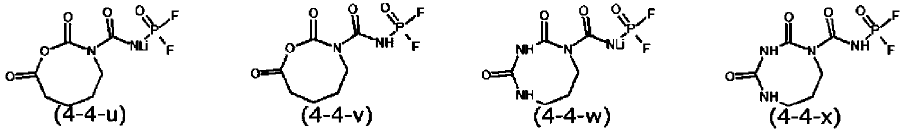

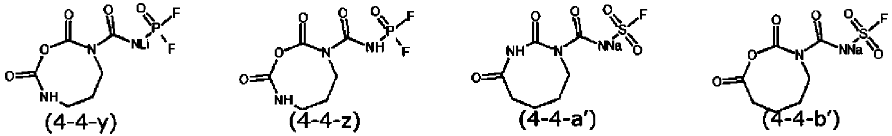

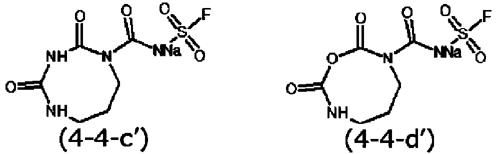

The compound represented by Formula (5) is described below.

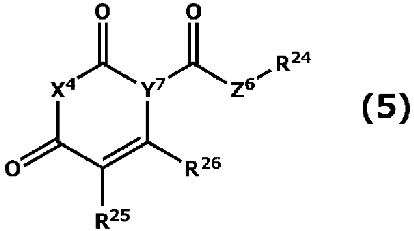

X⁴ represents CH₂, NH, O, or S, and Y⁷ represents CH or N. Z⁶ represents CH₂, O, or NR²⁷.

X⁴ may be NH or O.

Y⁷ may be N.

Z⁶ may be NR²⁷.

R²⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group. Any hydrogen atom of the alkyl group may be substituted with a fluorine atom. When R²⁷ represents an alkali metal cation, a bond between a nitrogen atom and R²⁷ represents an ionic bond.

R²⁷ may be a hydrogen atom, an alkali metal cation, a linear alkyl group having 1 to 4 carbon atoms, or a branched alkyl group having 3 to 4 carbon atoms, may be a hydrogen atom, a lithium ion, a sodium ion, a linear alkyl group having 1 to 4 carbon atoms, or a branched alkyl group having 3 to 4 carbon atoms, or may be a hydrogen atom, a lithium ion, a sodium ion, or a methyl group.

R²⁴ represents PO(R_{f})₂ or SO₂R_{f}.

R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group. Any hydrogen atom of the alkyl group, the alkenyl group, or the alkynyl group may be substituted with a fluorine atom. When there are a plurality of R_{f}, the plurality of R_{f} may be the same or different.

Specific examples of R_{f} representing a linear alkyl group having 1 to 12 carbon atoms or a branched alkyl group having 3 to 12 carbon atoms include a methyl group, an ethyl group, an n-propyl group, an isopropyl group, an n-butyl group, a sec-butyl group, an isobutyl group, a tert-butyl group, and an n-pentyl group.

Specific examples of R_{f} representing a linear alkenyl group having 2 to 12 carbon atoms or a branched alkenyl group having 3 to 12 carbon atoms include a vinyl group, an allyl group, a 1-propenyl group, an isopropenyl group, a 2-butenyl group, and a 1,3-butadienyl group.

Specific examples of R_{f} representing a linear alkynyl group having 2 to 12 carbon atoms or a branched alkynyl group having 4 to 12 carbon atoms include an ethynyl group, a 2-propynyl group, and a 1,1-dimethyl-2-propynyl group.

At least one of an oxygen atom and a nitrogen atom may be included between a carbon atom-carbon atom bond in the alkyl group, the alkenyl group, or the alkynyl group. Examples include a 2-methoxyethyl group, a 2-ethoxyethyl group, an (allyloxy)methyl group, a (propargyloxy)methyl group, a (dimethylamino)ethyl group, and a (diethylamino)ethyl group.

Any hydrogen atom of the alkyl group, the alkenyl group, or the alkynyl group may be substituted with a fluorine atom. Examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a 1,1-bis(trifluoromethyl)-2-propenyl group, and a 3,3,3-trifluoropropynyl group.

R²⁴ may be POF₂ or SO₂F, or may be SO₂F.

R²⁵ and R²⁶ each independently represent a halogen atom, a hydrogen atom, a nitro group, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R²⁵ and R²⁶ may each independently represent a halogen atom, a hydrogen atom, a nitro group, or an alkyl group having 1 to 4 carbon atoms, may be a fluorine atom, a hydrogen atom, a nitro group, or a methyl group, or may be a fluorine atom, a hydrogen atom, or a nitro group.

Specifically, the compound represented by Formula (5) may be at least one selected from the group consisting of compounds represented by the following Formulas (5-1-a) to (5-1-1).

At least one selected from the group consisting of the compound represented by Formula (5-1-e) (also referred to as a compound (5-1-e)), the compound represented by Formula (5-1-m) (also referred to as a compound (5-1-m)), the compound represented by Formula (5-1-o) (also referred to as a compound ((5-1-o)), the compound represented by Formula (5-1-y) (also referred to as a compound (5-1-y)), and the compound represented by Formula (5-1-d') (also referred to as a compound (5-1-d')) is more preferred.

At least one selected from the group consisting of the compound (5-1-e), the compound (5-1-m), and the compound (5-1-d') is furthermore preferred.

At least one selected from the group consisting of the compound (5-1-e) and the compound (5-1-m) is particularly preferred.

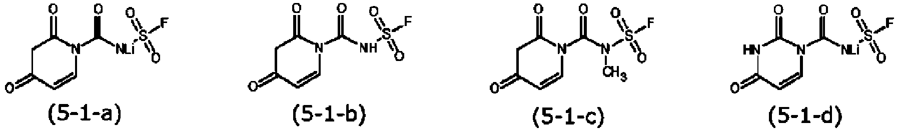

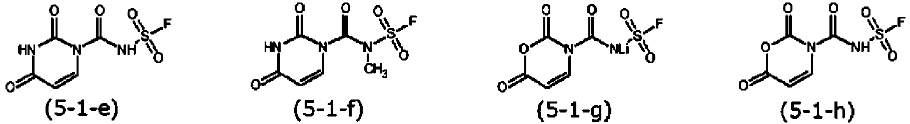

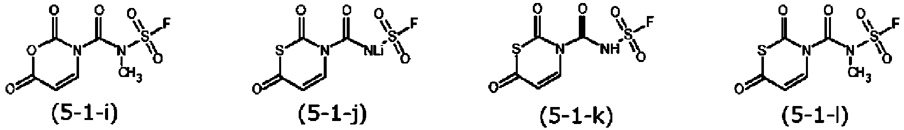

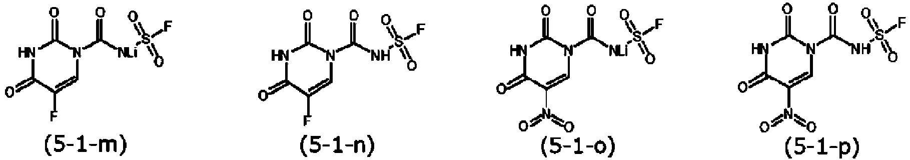

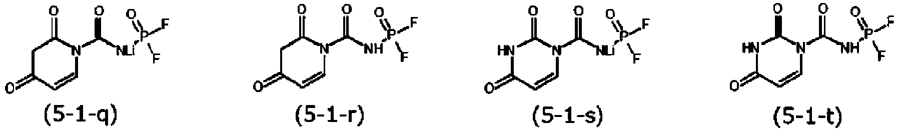

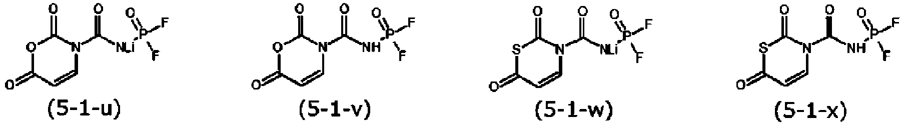

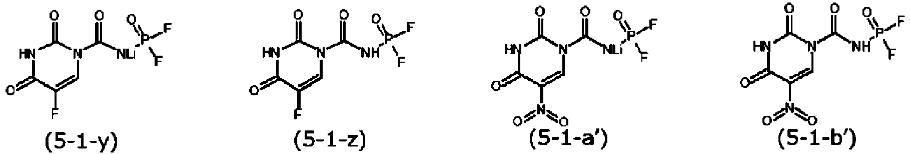

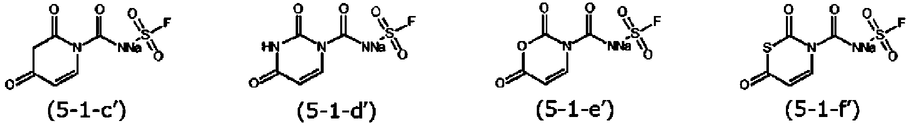

The compound represented by Formula (6) is described below.

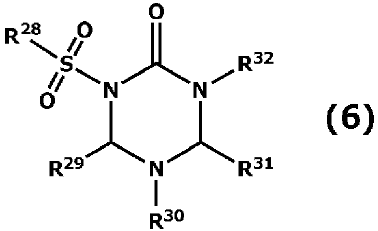

R²⁸ represents a halogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance when a film is formed on an electrode. The above alkyl group may be an alkyl group having 4 or less carbon atoms, and may be a methyl group, an ethyl group, an n-propyl group, an isopropyl group, an n-butyl group, or a tert-butyl group.

R²⁸ may be a halogen atom or an alkyl group having 1 to 4 carbon atoms, may be a fluorine atom or a methyl group, or may be a fluorine atom.

R²⁹ and R³¹ each independently represent a halogen atom, a hydrogen atom, a linear alkyl group having 1 to 12 carbon atoms, or a branched alkyl group having 3 to 12 carbon atoms. Specific examples of the alkyl group include a methyl group, an ethyl group, an n-propyl group, an isopropyl group, an n-butyl group, a sec-butyl group, an isobutyl group, a tert-butyl group, and an n-pentyl group.

An oxygen atom may be included between a carbon atom-carbon atom bond in the above alkyl group, and examples include a 2-methoxyethyl group and a 2-ethoxyethyl group.

Any hydrogen atom in the above alkyl group may be substituted with a fluorine atom, and examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, and a hexafluoroisopropyl group.

When the above alkyl group is an alkyl group having 6 or less carbon atoms, such an alkyl group can reduce resistance in the formation of a film on an electrode. The above alkyl group may be an alkyl group having 4 or less carbon atoms, and may be a methyl group, an ethyl group, an n-propyl group, an isopropyl group, an n-butyl group, or a tert-butyl group.

R²⁹ to R³¹ may each independently represent a halogen atom, a hydrogen atom, or an alkyl group having 1 to 4 carbon atoms, may be a hydrogen atom or a methyl group, or may be a hydrogen atom.

R³⁰ and R³² each independently represent a hydrogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms.

Specific examples of the alkyl group include a methyl group, an ethyl group, an n-propyl group, an isopropyl group, an n-butyl group, a sec-butyl group, an isobutyl group, a tert-butyl group, and an n-pentyl group.

Specific examples of the alkenyl group include an allyl group.

Specific examples of the alkynyl group include a propargyl group.

At least one of an oxygen atom and a nitrogen atom may be included between a carbon atom-carbon atom bond in the alkyl group, the alkenyl group, or the alkynyl group, and examples include a 2-methoxyethyl group, a 2-ethoxyethyl group, an (allyloxy)methyl group, a (propargyloxy)methyl group, a (dimethylamino)ethyl group, and a (diethylamino)ethyl group.

A carbon-nitrogen unsaturated bond may be included in the alkyl group, and examples include a cyanomethyl group and a cyanoethyl group.

Any hydrogen atom of the alkyl group, the alkenyl group, or the alkynyl group may be substituted with a fluorine atom. Examples include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a 1,1-bis(trifluoromethyl)-2-propenyl group, and a 3,3,3-trifluoropropynyl group.

If the alkyl group, the alkenyl group, or the alkynyl group preferably has 6 or less carbon atoms, resistance when a film is formed on an electrode can be reduced. The alkyl group, the alkenyl group, or the alkynyl group may have 4 or less carbon atoms, and may be a methyl group, an ethyl group, an n-propyl group, an isopropyl group, an n-butyl group, a tert-butyl group, an allyl group, a propargyl group, or a cyanomethyl group.

R³⁰ and R³² may each independently represent an alkyl group, an alkenyl group, or an alkynyl group having 1 to 4 carbon atoms, may be a methyl group, an ethyl group, an allyl group, a propargyl group, or a cyanomethyl group, or may be an allyl group, a propargyl group, or a cyanomethyl group.

Specifically, the compound represented by Formula (6) may be at least one selected from the group consisting of compounds represented by the following Formulas (6-1-a) to (6-1-j).

At least one selected from the group consisting of the compound represented by Formula (6-1-a) (also referred to as a compound (6-1-a)), the compound represented by Formula (6-1-c) (also referred to as a compound (6-1-c)), the compound represented by Formula (6-1-d) (also referred to as a compound ((6-1-d)), and the compound represented by Formula (6-1-e) (also referred to as a compound (6-1-e)) is more preferred.

At least one selected from the group consisting of the compound (6-1-c), the compound (6-1-d), and the compound (6-1-e) is furthermore preferred.

At least one selected from the group consisting of the compound (6-1-c) and the compound (6-1-e) is particularly preferred.

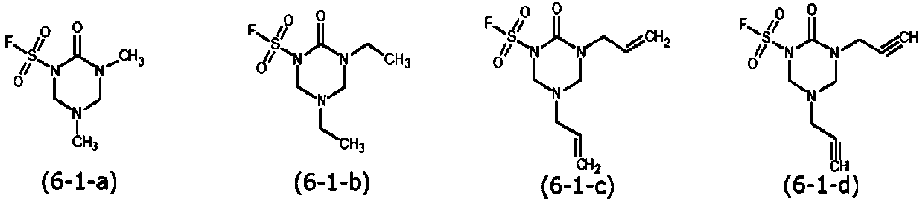

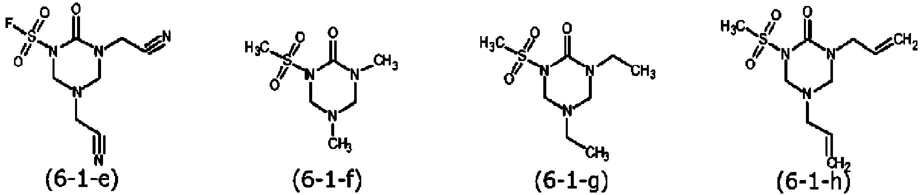

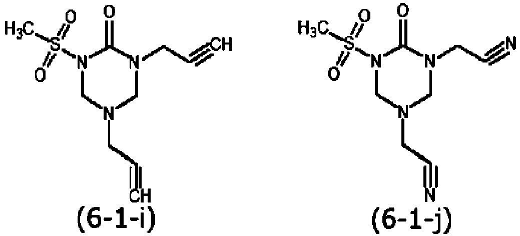

In the nonaqueous electrolyte solution according to the present disclosure, a lower limit of a total amount of the above component (I) (hereafter, also referred to as "concentration of (I)") with respect to a total amount (100% by mass) of the nonaqueous electrolyte solution may be set to 0.01% by mass or more, may be set to 0.05% by mass or more, and may be set to 0.1% by mass or more. An upper limit of the concentration of (I) may be set to 10.0% by mass or less, may be set to 5.0% by mass or less, or may be set to 2.0% by mass or less.

By setting the concentration of (I) to 0.01% by mass or more, an effect of preventing an increase in the initial resistance of the nonaqueous electrolyte solution battery using the nonaqueous electrolyte solution is easily exhibited. On the other hand, by setting the concentration of (I) to 10.0% by mass or less, an increase in viscosity of the nonaqueous electrolyte solution is prevented, and the effect of preventing the increase in resistance of the nonaqueous electrolyte solution battery using the nonaqueous electrolyte solution can be easily achieved.

In the nonaqueous electrolyte solution according to the present disclosure, one type of compound in the component (I) may be used alone, or two or more types of the compounds may be mixed and used in any combination and any proportion according to use.

The compound represented by Formula (1) can be produced by various methods. The production method is not particularly limited.

For example, the compound represented by Formula (1) can be obtained by allowing tetrahydro-2-pyrimidinone or 2-piperidone to react with fluorosulfonyl isocyanate or difluorophosphoryl isocyanate, followed by a reaction with lithium hydride, sodium hydride, or dialkyl sulfate.

The compound represented by Formula (2) can be produced by various methods. The production method is not particularly limited.

For example, the compound represented by Formula (2) can be obtained by allowing tetrahydro-2-pyrimidinone to react with fluorosulfonyl isocyanate or difluorophosphoryl isocyanate, followed by a reaction with lithium hydride, sodium hydride, or dialkyl sulfate.

The compound represented by Formula (3) can be produced by various methods. The production method is not particularly limited.

For example, the compound represented by Formula (3) can be obtained by allowing 1-methanesulfonyl-2-imidazolidinone or 1-fluorosulfonyl-2-imidazolidinone to react with fluorosulfonyl isocyanate or difluorophosphoryl isocyanate, followed by a reaction with lithium hydride, sodium hydride, or dialkyl sulfate.

The compound represented by Formula (4-1), the compound represented by Formula (4-2), or the compound represented by Formula (4-3) can be produced by various methods. The production method is not particularly limited.

For example, the compound represented by Formula (4-1), Formula (4-2), or Formula (4-3) can be obtained by allowing urazole or 2,5-oxazolidinedione to react with fluorosulfonyl isocyanate or difluorophosphoryl isocyanate, followed by a reaction with lithium hydride, sodium hydride, or dialkyl sulfate.

The compound represented by Formula (5) can be produced by various methods. The production method is not particularly limited.

For example, the compound represented by Formula (2) can be obtained by allowing uracil or fluorouracil to react with fluorosulfonyl isocyanate or difluorophosphoryl isocyanate, followed by a reaction with lithium hydride, sodium hydride, or dialkyl sulfate.

The compound represented by Formula (6) can be produced by various methods. The production method is not particularly limited.

For example, the compound represented by Formula (6) can be obtained by allowing methyleneaminoacetonitrile or methyleneaminopropene to react with fluorosulfonyl isocyanate or methanesulfonyl isocyanate.

The present disclosure relates to
a compound in which R¹ represents POF₂ or SO₂F in the above Formula (1);
a compound in which R⁶ and R⁷ each independently represent POF₂ or SO₂F in the above Formula (2);
a compound in which R¹² represents POF₂ or SO₂F in the above Formula (3);
a compound in which R¹⁸ represents POF₂ or SO₂F in the above Formulas (4-1), (4-2), and (4-3);
a compound in which X⁴ represents CH₂, NH, or O and R²⁴ represents POF₂ or SO₂F in the above Formula (5); and
a compound in which R²⁸ represents a fluorine atom in the above Formula (6).

The above compounds are suitably used as an additive in a nonaqueous electrolyte solution.

### <(II) Solute>

The nonaqueous electrolyte solution according to the present disclosure contains the solute.

The solute is not limited, but may be an ionic salt, or may be an ionic salt containing fluorine.

The solute may be, for example, an ionic salt containing a pair of at least one cation selected from the group consisting of alkali metal ions such as lithium ions and sodium ions, alkaline earth metal ions, and quaternary ammonium, and at least one anion selected from the group consisting of a hexafluorophosphate anion, a tetrafluoroborate anion, a perchlorate anion, a hexafluoroarsenate anion, a hexafluoroantimonate anion, a perfluoroalkanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, a tris(trifluoromethanesulfonyl)methide anion, an aluminate anion, a chloroaluminate anion, and a halogen anion.

The solute may be at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

One type of these solutes may be used alone, or two or more types thereof may be mixed and used in any combination and any proportion according to use.

Among the above solutes, from a viewpoint of energy density, output characteristics, life, and the like of a nonaqueous electrolyte solution battery, the cation may be at least one selected from the group consisting of lithium, sodium, potassium, magnesium, and quaternary ammonium, and the anion may be at least one selected from the group consisting of a hexafluorophosphate anion, a tetrafluoroborate anion, a bis(trifluoromethanesulfonyl)imide anion, and a bis(fluorosulfonyl)imide anion.

A total amount of the solute in the nonaqueous electrolyte solution according to the present disclosure (hereinafter, also referred to as a "solute concentration") is not particularly limited, but a lower limit may be set to 0.5 mol/L or more, 0.7 mol/L or more, or 0.9 mol/L or more with respect to the total amount of the nonaqueous electrolyte solution. An upper limit of the solute concentration may be set to 5.0 mol/L or less, 4.0 mol/L or less, or 2.0 mol/L or less. By setting the solute concentration to 0.5 mol/L or more, the deterioration of the cycle characteristics and the output characteristics of the nonaqueous electrolyte solution battery due to the decrease in ionic conductivity can be suppressed, and by setting the solute concentration to 5.0 mol/L or less, the deterioration in ionic conductivity, and the deterioration in the cycle characteristics and the output characteristics of the nonaqueous electrolyte solution battery due to an increase in viscosity of the nonaqueous electrolyte solution can be suppressed.

### <(III) Nonaqueous Organic Solvent>

A type of the nonaqueous organic solvent used in the nonaqueous electrolyte solution according to the present disclosure is not limited, and any nonaqueous organic solvent can be used.

The nonaqueous organic solvent may contain at least one selected from the group consisting of a cyclic ester, a chain ester, a cyclic ether, a chain ether, a sulfone compound, a sulfoxide compound, and an ionic liquid.

Specifically, the nonaqueous organic solvent may contain at least one selected from the group consisting of ethyl methyl carbonate (hereinafter, also referred to as "EMC"), dimethyl carbonate (hereinafter, also referred to as "DMC"), diethyl carbonate (hereinafter, also referred to as "DEC"), methyl propyl carbonate, ethyl propyl carbonate, methyl butyl carbonate, methyl 2,2,2-trifluoroethyl carbonate, ethyl 2,2,2-trifluoroethyl carbonate, propyl 2,2,2-trifluoroethyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter, also referred to as "EC"), propylene carbonate (hereinafter, also referred to as "PC"), butylene carbonate, fluoroethylene carbonate (hereinafter, also referred to as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethyl sulfoxide, sulfolane, γ-butyrolactone, and γ-valerolactone.

In the present disclosure, an ionic liquid having a salt structure may be used as the nonaqueous organic solvent.

If the nonaqueous organic solvent contains at least one selected from the group consisting of a cyclic ester and a chain ester, input and output characteristics at low temperatures are excellent.

If the cyclic ester is cyclic carbonate, cycle characteristics at high temperatures are excellent.

Specific examples of the above cyclic carbonate include EC, PC, butylene carbonate, and FEC, and at least one selected from the group consisting of EC, PC, and FEC is preferred.

If the chain ester is chain carbonate, cycle characteristics at high temperatures are excellent.

Specific examples of the above chain carbonate include EMC, DMC, DEC, methyl propyl carbonate, ethyl propyl carbonate, methyl 2,2,2-trifluoroethyl carbonate, ethyl 2,2,2-trifluoroethyl carbonate, 1,1,1,3,3,3-hexafluoro-1-propylmethyl carbonate, and 1,1,1,3,3,3-hexafluoro-1-propylethyl carbonate, and at least one selected from the group consisting of EMC, DMC, DEC, and methyl propyl carbonate is preferred.

Specific examples of the above ester include methyl acetate, ethyl acetate, methyl propionate, ethyl propionate, methyl 2-fluoropropionate, and ethyl 2-fluoropropionate.

### <Other Additives>

An additive component to be commonly used in the nonaqueous electrolyte solution according to the present disclosure may be further added in any proportion as long as the gist of the present disclosure is not impaired.

Specific examples of other additives include compounds having an overcharge prevention effect, a negative electrode film-forming effect, and a protective effect of a positive electrode, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl propargyl carbonate, ethyl propargyl carbonate, dipropargyl carbonate, maleic anhydride, succinic anhydride, propanesultone, 1,3-propanesultone, 1,3-propenesultone, butanesultone, 1,3,2-dioxathiolane-2,2-dioxide, 4-propyl-1,3,2-dioxathiolane-2,2-dioxide, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, sodium tris(oxalato)phosphate, potassium tris(oxalato)phosphate, lithium difluorophosphate, sodium difluorophosphate, potassium difluorophosphate, lithium monofluorophosphate, sodium monofluorophosphate, potassium monofluorophosphate, lithium fluorosulfonate, sodium fluorosulfonate, potassium fluorosulfonate, lithium bis(difluorophosphoryl)imide, sodium bis(difluorophosphoryl)imide, potassium bis(difluorophosphoryl)imide, lithium (difluorophosphoryl)(fluorosulfonyl)imide, sodium (difluorophosphoryl)(fluorosulfonyl)imide, potassium (difluorophosphoryl)(fluorosulfonyl)imide, methanesulfonyl fluoride, ethenesulfonyl fluoride, and phenyl difluorophosphate, and these compounds can be used alone or in combination of two or more kinds thereof.

The nonaqueous electrolyte solution according to the present disclosure may contain at least one selected from vinylene carbonate, bis(oxalato)borate, difluorooxalatoborate, difluorobis(oxalato)phosphate, tetrafluorooxalatophosphate, tetrafluoro(malonato)phosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(picolinato)phosphate, 1,3-dimethyl-1,3-divinyl-1,3-di(1,1,1,3,3,3-hexafluoroisopropyl)disiloxane, t-butylbenzene, t-amylbenzene, fluorobenzene, and cyclohexylbenzene.

A content of the above additive in the nonaqueous electrolyte solution may be 0.01% by mass or more and 5.0% by mass or less with respect to the total amount of the nonaqueous electrolyte solution.

The nonaqueous electrolyte solution according to the present disclosure may contain a compound represented by the following Formula (7) as other additives.

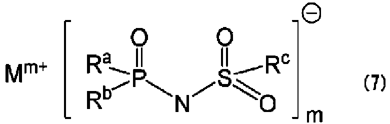

[In Formula (7), R^{a} to R^{c} are each independently a fluorine atom or an organic group selected from a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group optionally has a fluorine atom, an oxygen atom, and/or an unsaturated bond. Here, at least one of R^{a} to R^{c} is a fluorine atom.

M^{m+} represents an alkali metal cation, an alkaline earth metal cation, or an onium cation, and m represents the same integer as a valence of the corresponding cation.]

When the compound (salt having an imide anion) represented by Formula (7) has at least one P-F bond or S-F bond, the compound has excellent low-temperature characteristics. The greater the number of P-F bonds or S-F bonds in the salt having an imide anion, the more the low-temperature characteristics can be improved. Therefore, the salt having an imide anion represented by the above Formula (7) may be a compound in which all of R^{a} to R^{c} are fluorine atoms.

The salt having an imide anion represented by the above Formula (7) may be a compound in which at least one of R^{a} to R^{c} is a fluorine atom, and at least one of R^{a} to R^{c} is selected from a hydrocarbon group having 6 or less carbon atoms and optionally having a fluorine atom.

The salt having an imide anion represented by the above Formula (7) may be a compound in which at least one of R^{a} to R^{c} is a fluorine atom, and at least one of R^{a} to R^{c} is selected from a methyl group, a methoxy group, an ethyl group, an ethoxy group, a propyl group, a propoxy group, a vinyl group, an allyl group, an allyloxy group, an ethynyl group, a 2-propynyl group, a 2-propynyloxy group, a phenyl group, a phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

A counter cation M^{m+} of the salt having an imide anion represented by the above Formula (7) may be selected from the group consisting of a lithium ion, a sodium ion, a potassium ion, and a tetraalkylammonium ion.

In the above Formula (7), examples of the alkyl group and the alkoxy group represented by R^{a} to R^{c} include alkyl groups having 1 to 10 carbon atoms such as a methyl group, an ethyl group, a propyl group, an isopropyl group, a butyl group, a sec-butyl group, a tert-butyl group, a pentyl group, a 2,2-difluoroethyl group, a 2,2,2-trifluoroethyl group, a 2,2,3,3-tetrafluoropropyl group, and a 1,1,1,3,3,3-hexafluoroisopropyl group, fluorine-containing alkyl groups, and alkoxy groups derived from these groups.

Examples of the alkenyl group and the alkenyloxy group include alkenyl groups having 2 to 10 carbon atoms such as a vinyl group, an allyl group, a 1-propenyl group, an isopropenyl group, a 2-butenyl group, and a 1,3-butadienyl group, fluorine-containing alkenyl groups, and alkenyloxy groups derived from these groups.

Examples of the alkynyl group and the alkynyloxy group include an alkynyl group having 2 to 10 carbon atoms such as an ethynyl group, a 2-propynyl group, and a 1,1-dimethyl-2-propynyl group, fluorine-containing alkynyl groups, and alkynyloxy groups derived from these groups.

Examples of the cycloalkyl group and the cycloalkoxy group include a cycloalkyl group having 3 to 10 carbon atoms such as a cyclopentyl group and a cyclohexyl group, fluorine-containing cycloalkyl groups, and cycloalkoxy groups derived from these groups.

Examples of the cycloalkenyl group and the cycloalkenyloxy group include a cycloalkenyl group having 3 to 10 carbon atoms such as a cyclopentenyl group and a cyclohexenyl group, fluorine-containing cycloalkenyl groups, and cycloalkenyloxy groups derived from these groups.

Examples of the aryl group and the aryloxy group include an aryl group having 6 to 10 carbon atoms such as a phenyl group, a tolyl group, and an xylyl group, fluorine-containing aryl groups, and aryloxy groups derived from these groups.

Specific examples and synthesis methods of the salt having the imide anion represented by the above Formula (7) include those described in WO2017/111143.

A content of the salt having an imide anion represented by the above Formula (7) in the nonaqueous electrolyte solution may be 0.01% by mass or more and 8.0% by mass or less with respect to the total amount of the nonaqueous electrolyte solution.

When a content of the ionic salt exemplified as the solute is less than 0.5 mol/L, which is the lower limit of the suitable concentration of the solute, in the nonaqueous electrolyte solution, the ionic salt can exert a negative electrode film-forming effect and a protective effect of a positive electrode as "other additives". In this case, the content in the nonaqueous electrolyte solution may be 0.01% by mass to 5.0% by mass.

For example, when the nonaqueous electrolyte solution battery is a lithium ion battery, examples of the ionic salt in this case include lithium hexafluorophosphate, lithium tetrafluoroborate, lithium trifluoromethanesulfonate, lithium bis(trifluoromethanesulfonyl)imide, lithium bis(fluorosulfonyl)imide, and lithium (trifluoromethanesulfonyl)(fluorosulfonyl)imide, and when the nonaqueous electrolyte solution battery is a sodium ion battery, examples of the ionic salt include sodium hexafluorophosphate, sodium tetrafluoroborate, sodium trifluoromethanesulfonate, sodium bis(trifluoromethanesulfonyl)imide, sodium bis(fluorosulfonyl)imide, and sodium (trifluoromethanesulfonyl)(fluorosulfonyl)imide.

An alkali metal salt other than the above solutes may be used as an additive.

Specific examples include carboxylates such as lithium acrylate, sodium acrylate, lithium methacrylate, and sodium methacrylate, and sulfate ester salts such as lithium methyl sulfate, sodium methyl sulfate, lithium ethyl sulfate, and sodium ethyl sulfate.

From a viewpoint of improving durability (life) of a battery, when the nonaqueous electrolyte solution battery is a lithium ion battery, the nonaqueous electrolyte solution according to the present disclosure may contain, with respect to the total amount of the nonaqueous electrolyte solution, 0.01% to 5.0% by mass of at least one selected from vinylene carbonate, fluoroethylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium (difluorophosphoryl)(fluorosulfonyl)imide, lithium difluorophosphate, lithium fluorosulfonate, 1,3-propenesultone, 1,3-propanesultone, 1,3,2-dioxathiolane-2,2-dioxide, and 4-propyl-1,3,2-dioxathiolane-2,2-dioxide among the above other additives.

When the nonaqueous electrolyte solution battery is a sodium ion battery, the nonaqueous electrolyte solution according to the present disclosure may contain, with respect to the total amount of the nonaqueous electrolyte solution, 0.01% to 5.0% by mass of at least one selected from vinylene carbonate, fluoroethylene carbonate, sodium bis(oxalato)borate, sodium difluorooxalatoborate, sodium difluorobis(oxalato)phosphate, sodium tetrafluorooxalatophosphate, sodium bis(fluorosulfonyl)imide, sodium (difluorophosphoryl)(fluorosulfonyl)imide, sodium difluorophosphate, sodium fluorosulfonate, 1,3-propenesultone, 1,3-propanesultone, 1,3,2-dioxathiolane-2,2-dioxide, and 4-propyl-1,3,2-dioxathiolane-2,2-dioxide.

In addition, the nonaqueous electrolyte solution according to the present disclosure may also contain a polymer, and as in the case of being used in a nonaqueous electrolyte solution battery referred to as a polymer battery, the nonaqueous electrolyte solution can be quasi-solidified with a gelling agent or a cross-linked polymer. The polymer solid electrolyte includes one containing the nonaqueous organic solvent as a plasticizer.

The above polymer is not limited as long as the polymer is an aprotic polymer capable of dissolving the above (I) and (II) and the above other additives. Examples of the above polymer include polymers having polyethylene oxide as a main chain or a side chain, homopolymers or copolymers of polyvinylidene fluoride, methacrylic acid ester polymers, and polyacrylonitrile. When the plasticizer is added to such a polymer, among the above nonaqueous organic solvents, an aprotic nonaqueous organic solvent may be used.

### [2. Nonaqueous Electrolyte Solution Battery]

The nonaqueous electrolyte solution battery according to the present disclosure includes, at least, the nonaqueous electrolyte solution according to the present disclosure described above, a negative electrode, a positive electrode, and a separator. An exterior body or the like may be further included.

The negative electrode is not limited, but may use a material capable of reversibly intercalating and deintercalating alkali metal ions such as lithium ions and sodium ions, or alkaline earth metal ions.

For example, in the case of the lithium ion secondary battery in which cations are mainly lithium, a negative electrode active material constituting the negative electrode is capable of doping and dedoping lithium ions. Examples of the negative electrode active material include a material containing at least one selected from, for example, a carbon material in which a d value of a lattice plane (002 plane) in X-ray diffraction is 0.340 nm or less, a carbon material in which the d value of the lattice plane (002 plane) in X-ray diffraction exceeds 0.340 nm, oxides of one or more metal selected from Si, Sn, and Al, one or more metals selected from Si, Sn, and Al, alloys containing these metals, alloys of these metals and lithium, or alloys of the above alloys and lithium, and lithium titanium oxide. One of these negative electrode active materials can be used alone, or two or more thereof can be used in combination. Lithium metal, metal nitrides, tin compounds, conductive polymers, and the like may also be used.

For example, in the case of a sodium ion secondary battery in which a cation is mainly sodium, as the negative electrode active material constituting the negative electrode, sodium metal, an alloy of sodium metal and other metals such as tin, an intermetallic compound, various carbon materials such as hard carbon, a metal oxide such as titanium oxide, a metal nitride, (elemental) tin, a tin compound, activated carbon, a conductive polymer, and the like may be used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like may be used. One type of these negative electrode active materials may be used alone, or two or more types thereof may be used in combination.

The positive electrode is not limited, but may use a material capable of reversibly intercalating and deintercalating alkali metal ions such as lithium ions and sodium ions, or alkaline earth metal ions.

For example, when the cation is lithium, lithium-containing transition metal oxide composites such as LiCoO₂, LiNiO₂, LiMnO₂, and LiMn₂O₄, lithium-containing transition metal oxide composites including a mixture of a plurality of transition metals in the lithium-containing transition metal oxide composites such as Co, Mn, and Ni, lithium-containing transition metal oxide composites in which a part of the transition metals in the lithium-containing transition metal oxide composites is substituted with a metal other than the transition metals, phosphate compounds of transition metals such as LiFePO₄, LiCoPO₄, and LiMnPO₄ referred to as olivine, oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material.

For example, when the cation is sodium, sodium-containing transition metal oxide composites such as NaCrO₂, NaFe_{0.3}Co_{0.3}O₂, NaFe_{0.4}Mn_{0.3}Ni_{0.3}O₂, NaNi_{0.3}Ti_{0.3}Mn_{0.2}O₂, NaNi_{1/3}Ti_{1/3}Mn_{1/3}O₂, NaNi_{0.33}Ti_{0.33}Mn_{0.16}Mg_{0.17}O₂, Na_{2/3}Ni_{1/3}Ti_{1/6}Mn_{1/2}O₂, and Na_{2/3}Ni_{1/3}Mn_{2/3}O₂, sodium-containing transition metal oxide composites including a mixture of a plurality of transition metals in the sodium-containing transition metal oxide composites such as Co, Mn, and Ni, sodium-containing transition metal oxide composites in which a part of the transition metals in the sodium-containing transition metal oxide composites is substituted with a metal other than the transition metals, polyanion type compounds such as NaFePO₄, NaVPO₄F, Na₃V₂(PO₄)₃, and Na₂Fe₂(SO₄)₃, sodium salts of Prussian Blue analogues represented by a composition: NaₐM₃[Fe(CN)₆]_{c} (M = Cr, Mn, Fe, Co, Ni, Cu, or Zn, 0 ≤ a ≤ 2, 0.5 ≤ b ≤ 1.5, 0.5 ≤ c ≤ 1.5), oxides such as TiO₂, V₂O₃, and MoO₃, sulfides such as TiS₂ and FeS, conductive polymers such as polyacetylene, polyparaphenylene, polyaniline, and polypyrrole, activated carbon, radical-generating polymers, carbon materials, and the like may be used as a positive electrode material (positive electrode active material).

An electrode sheet obtained by mixing acetylene black, ketjen black, carbon fiber, or graphite as a conductive material, and polytetrafluoroethylene, polyvinylidene fluoride, SBR resin, or the like as a binder and molding the mixture into a sheet shape can be used to the positive electrode material and negative electrode material.

As a separator for preventing contact between the positive electrode and the negative electrode, a nonwoven fabric or porous sheet made of polypropylene, polyethylene, paper, glass fiber, or the like may be used.

An electrochemical device having a shape such as a coin shape, a cylindrical shape, a square shape, or an aluminum laminate sheet shape is assembled based on the above elements.

### Examples

Hereinafter, the present disclosure will be described in more detail below with reference to Examples, but the present disclosure is not limited by these descriptions.

### <Synthesis Example 1>

After 10.0 g of EMC and 0.5 g of tetrahydro-2-pyrimidinone were put in a 50 mL eggplant flask, 0.6 g of fluorosulfonyl isocyanate was slowly added. After an internal temperature decreased to 25°C, 0.04 g of lithium hydride was added. After foaming ceased, concentration was carried out to obtain 1.1 g of compound (1-1-g) (yield: 98%).
¹H NMR(CD₃CN) σ_{H} 3.90, 3.72, 2.20ppm, ¹⁹F NMR(CD₃CN) σ_{f} 49.5ppm.

### <Synthesis Example 2>

After 10.0 g of EMC and 0.6 g of hexahydro-1,3-diazepin-2-one were put in a 50 mL eggplant flask, 0.6 g of fluorosulfonyl isocyanate was slowly added. After an internal temperature decreased to 25°C, 0.04 g of lithium hydride was added. After foaming ceased, concentration was carried out to obtain 1.4 g of compound (1-2-g) (yield: 96%).
¹H NMR(CD₃CN) σ_{H} 3.82, 3.40, 3.01, 2.41ppm, ¹⁹F NMR(CD₃CN) σ_{F} 49.7ppm.

### <Synthesis Example 3>

After 10.0 g of EMC and 0.5 g of tetrahydro-2-pyrimidinone were put in a 50 mL eggplant flask, 1.3 g of fluorosulfonyl isocyanate was slowly added. After an internal temperature was lowered to 25°C, concentration was carried out to obtain 1.8 g of compound (2-1-d) (yield: 98%).
¹H NMR(CD₃CN) σ_{H} 12.0, 3.83, 1.95ppm, ¹⁹F NMR(CD₃CN) σ_{F} 50.9ppm.

### <Synthesis Example 4>

After 10.0 g of EMC and 0.6 g of hexahydro-1,3-diazepin-2-one were put in a 50 mL eggplant flask, 1.3 g of fluorosulfonyl isocyanate was slowly added. After an internal temperature decreased to 25°C, 0.08 g of lithium hydride was added. After foaming ceased, concentration was carried out to obtain 1.4 g of compound (2-2-a) (yield: 97%).
¹H NMR(CD₃CN) σ_{H} 3.91, 3.10ppm, ¹⁹F NMR(CD₃CN) σ_{F} 50.6ppm.

### <Synthesis Example 5>

After 10.0 g of EMC and 0.8 g of 1-methanesulfonyl-2-imidazolidinone were put in a 50 mL eggplant flask, 0.6 g of fluorosulfonyl isocyanate was slowly added. After an internal temperature was lowered to 25°C, concentration was carried out to obtain 1.4 g of compound (3-1-e) (yield: 97%).
¹H NMR(CD₃CN) σ_{H} 11.5, 3.70, 3.40ppm, ¹⁹F NMR(CD₃CN) σ_{F} 51.3ppm.

### <Synthesis Example 6>

After 10.0 g of EMC and 0.9 g of 1-methanesulfonyl-2-tetrahydropyrimidinone were put in a 50 mL eggplant flask, 0.6 g of fluorosulfonyl isocyanate was slowly added. After an internal temperature was lowered to 25°C, concentration was carried out to obtain 1.4 g of compound (3-2-e) (yield: 97%).
¹H NMR(CD₃CN) σ_{H} 11.3, 3.82, 3.74, 3.40, 2.08ppm, ¹⁹F NMR(CD₃CN) σ_{F} 51.1ppm.

### <Synthesis Example 7>

After 10.0 g of EMC and 0.5 g of urazole were put in a 50 mL eggplant flask, 0.6 g of fluorosulfonyl isocyanate was slowly added. After an internal temperature decreased to 25°C, 0.04 g of lithium hydride was added. After foaming ceased, concentration was carried out to obtain 1.0 g of compound (4-1-j) (yield: 96%).
¹H NMR(CD₃CN) σ_{H} 9.60,9.42ppm, ¹⁹F NMR(CD₃CN) σ_{F} 54.9ppm.

### <Synthesis Example 8>

After 10.0 g of EMC and 0.6 g of 1,3,5-triazinane-2,4-dione were put in a 50 mL eggplant flask, 0.6 g of fluorosulfonyl isocyanate was slowly added. After an internal temperature decreased to 25°C, 0.04 g of lithium hydride was added. After foaming ceased, concentration was carried out to obtain 1.0 g of compound (4-2-j) (yield: 83%).
¹H NMR(CD₃CN) σ_{H} 10.93, 9.52, 7.20ppm, ¹⁹F NMR(CD₃CN) σ_{F} 54.0ppm.

### <Synthesis Example 9>

After 10.0 g of EMC and 0.6 g of uracil were put in a 50 mL eggplant flask, 0.6 g of fluorosulfonyl isocyanate was slowly added. After an internal temperature was lowered to 25°C, concentration was carried out to obtain 1.0 g of compound (5-1-e) (yield: 81%).
¹H NMR(CD₃CN) σ_{H} 11.60, 11.20, 7.90, 5.54ppm, ¹⁹F NMR(CD₃CN) σ_{F} 49.2ppm.

### <Synthesis Example 10>

After 10.0 g of EMC and 0.3 g of methylene amino acetonitrile were put in a 50 mL eggplant flask, 0.3 g of fluorosulfonyl isocyanate was slowly added. After an internal temperature was lowered to 25°C, concentration was carried out to obtain 0.6 g of compound (6-1-e) (yield: 99%).
¹H NMR(CD₃CN) σ_{H} 5.02, 4.49, 4.28, 3.96ppm, ¹⁹F NMR(CD₃CN) σ_{F} 54.8ppm.

### [Preparation of Nonaqueous Electrolyte Solutions according to Examples and Comparative Examples]

### <Comparative Example 1-1>

### (Preparation of LiPF₆ Solution)

EC, FEC, EMC, and DMC were mixed in a volume ratio of EC:FEC:EMC:DMC = 2:1:3:4 in a glove box having a dew point of -60°C or lower (component (III)). After that, LiPF₆ (component (II)) was added such that LiPF₆ had a concentration of 1.0 mol/L with respect to the total amount of the nonaqueous electrolyte solution, with keeping an internal temperature at 40°C or lower. The mixture was stirred, and LiPF₆ was completely dissolved to obtain a LiPF₆ solution. This LiPF₆ solution was set as a comparative nonaqueous electrolyte solution 1-1.

### <Example 1-1>

### (Preparation of Nonaqueous Electrolyte Solution 1-1)

EC, FEC, EMC, and DMC were mixed in a volume ratio of EC:FEC:EMC:DMC = 2:1:3:4 in a glove box having a dew point of -60°C or lower (component (III)). After that, LiPF₆ (component (II)) was added such that LiPF₆ had a concentration of 1.0 mol/L with respect to the total amount of the nonaqueous electrolyte solution, with keeping the internal temperature at 40°C or lower, the compound (1-1-g) (component (I)) corresponding to the compound represented by Formula (1) was added such that the compound (1-1-g) had a concentration of 0.5% by mass with respect to the total amount of the nonaqueous electrolyte solution. The mixture was stirred for 1 hour for dissolution to obtain a nonaqueous electrolyte solution 1-1 of Example 1-1.

### <Examples 1-2 to 1-15 and Comparative Examples 1-2 to 1-5>

### (Preparation of Nonaqueous Electrolyte Solutions 1-2 to 1-15 and Comparative Nonaqueous Electrolyte Solutions 1-2 to 1-5)

Except that the type and content of the component (I) (or a comparative compound) were changed as described in Table 1, nonaqueous electrolyte solutions 1-2 to 1-15 and comparative nonaqueous electrolyte solutions 1-2 to 1-5 were obtained in the same way as the preparation of the nonaqueous electrolyte solution 1-1. In the following tables, MP means N-methylpyrrolidone, and DMI means 1,3-dimethylimidazolidinone.

### <Examples 2-1 to 2-10 and Comparative Examples 2-1 to 2-3>

### (Preparation of Nonaqueous Electrolyte Solutions 2-1 to 2-10 and Comparative Nonaqueous Electrolyte Solutions 2-1 to 2-3)

Furthermore, except that vinylene carbonate (hereinafter, also referred to as "VC") as other additives was added and dissolved such that vinylene carbonate had a concentration described in Table 2, nonaqueous electrolyte solutions 2-1 to 2-10 and comparative nonaqueous electrolyte solutions 2-1 to 2-3 were respectively obtained in the same way as in the preparation of the nonaqueous electrolyte solutions 1-2, 1-4, 1-6, 1-8, 1-10, 1-11, 1-12, 1-13, 1-14, and 1-15 and the comparative nonaqueous electrolyte solutions 1-1, 1-3, and 1-5.

### <Examples 3-1 to 3-10 and Comparative Examples 3-1 to 3-3>

### (Preparation of Nonaqueous Electrolyte Solutions 3-1 to 3-10 and Comparative Nonaqueous Electrolyte Solutions 3-1 to 3-3)

Except that VC was changed to lithium bis(oxalato)borate (hereinafter, also referred to as "BOB"), nonaqueous electrolyte solutions 3-1 to 3-10 and comparative nonaqueous electrolyte solutions 3-1 to 3-3 were respectively obtained in the same way as the preparation of the nonaqueous electrolyte solutions 2-1 to 2-10 and the comparative nonaqueous electrolyte solutions 2-1 to 2-3.

### <Examples 4-1 to 4-10 and Comparative Examples 4-1 to 4-3>

### (Preparation of Nonaqueous Electrolyte Solutions 4-1 to 4-10 and Comparative Nonaqueous Electrolyte Solutions 4-1 to 4-3)

Except that VC was changed to lithium difluorobis(oxalato)phosphate (hereinafter, also referred to as "DFBOP"), nonaqueous electrolyte solutions 4-1 to 4-10 and comparative nonaqueous electrolyte solutions 4-1 to 4-3 were respectively obtained in the same way as the preparation of the nonaqueous electrolyte solutions 2-1 to 2-10 and the comparative nonaqueous electrolyte solutions 2-1 to 2-3.

### <Examples 5-1 to 5-10 and Comparative Examples 5-1 to 5-3>

### (Preparation of Nonaqueous Electrolyte Solutions 5-1 to 5-10 and Comparative Nonaqueous Electrolyte Solutions 5-1 to 5-3)

Except that VC was changed to lithium tetrafluorooxalatophosphate (hereinafter, also referred to as "TFOP"), nonaqueous electrolyte solutions 5-1 to 5-10 and comparative nonaqueous electrolyte solutions 5-1 to 5-3 were respectively obtained in the same way as the preparation of the nonaqueous electrolyte solutions 2-1 to 2-10 and the comparative nonaqueous electrolyte solutions 2-1 to 2-3.

### <Examples 6-1 to 6-10 and Comparative Examples 6-1 to 6-3>

### (Preparation of Nonaqueous Electrolyte Solutions 6-1 to 6-10 and Comparative Nonaqueous Electrolyte Solutions 6-1 to 6-3)

Except that VC was changed to lithium bis(fluorosulfonyl)imide (hereinafter, also referred to as "FSI"), nonaqueous electrolyte solutions 6-1 to 6-10 and comparative nonaqueous electrolyte solutions 6-1 to 6-3 were respectively obtained in the same way as the preparation of the nonaqueous electrolyte solutions 2-1 to 2-10 and the comparative nonaqueous electrolyte solutions 2-1 to 2-3.

### <Examples 7-1 to 7-10 and Comparative Examples 7-1 to 7-3>

### (Preparation of Nonaqueous Electrolyte Solutions 7-1 to 7-10 and Comparative Nonaqueous Electrolyte Solutions 7-1 to 7-3)

Except that VC was changed to lithium difluorophosphate (hereinafter, also referred to as "DFP"), nonaqueous electrolyte solutions 7-1 to 7-10 and comparative nonaqueous electrolyte solutions 7-1 to 7-3 were respectively obtained in the same way as the preparation of the nonaqueous electrolyte solution 2-1 to 2-10 and the comparative nonaqueous electrolyte solutions 2-1 to 2-3.

### <Examples 8-1 to 8-10, Comparative Examples 8-1 to 8-3>

### (Preparation of nonaqueous electrolyte solutions 8-1 to 8-10 and Comparative nonaqueous electrolyte solutions 8-1 to 8-3)

Except that VC was changed to lithium fluorosulfonate (hereinafter, also referred to as "FS"), nonaqueous electrolyte solutions 8-1 to 8-10 and comparative nonaqueous electrolyte solutions 8-1 to 8-3 were respectively obtained in the same way as in the preparation of nonaqueous electrolyte solutions 2-1 to 2-10 and comparative nonaqueous electrolyte solutions 2-1 to 2-3.

In the following Tables 1 to 8, the content of the component (I) (or the comparative compound) represents the concentration with respect to the total amount of nonaqueous electrolyte solution. The content of other additives represents the concentration with respect to the total amount of the nonaqueous electrolyte solution.

### [Production of Nonaqueous Electrolyte Solution Battery]

### (Production of NCM622 Positive Electrode)

To 90.0% by mass of LiNi_{0.6}Co_{0.2}Mn_{0.2}O₂ powder, 5.0% by mass of polyvinylidene fluoride (hereinafter, also referred to as PVDF) as a binder and 5.0% by mass of acetylene black as a conductive material were mixed, and N-methyl-2-pyrrolidone was further added to produce a positive electrode mixture paste. The paste was applied onto both sides of aluminum foil (A1085), and the resultant product was dried and pressed, and then punched into 4 cm × 5 cm pieces to obtain an NCM622 positive electrode for testing.

### (Production of NCM811 Positive Electrode)

To 92.0% by mass of LiNi_{0.8}Co_{0.1}Mn_{0.1}O₂ powder, 3.5% by mass of PVDF as a binder and 4.5% by mass of acetylene black as a conductive material were mixed, and N-methyl-2-pyrrolidone was further added to produce a positive electrode mixture paste. The paste was applied onto both sides of aluminum foil (A1085), and the resultant product was dried and pressed, and then punched into 4 cm × 5 cm pieces to obtain an NCM811 positive electrode for testing.

### (Production of Artificial Graphite Negative Electrode)

To produce a negative electrode mixture paste, 90.0% by mass of artificial graphite powder, 5.0% by mass of PVDF as a binder, and 5.0% by mass of acetylene black as a conductive material were mixed. The paste was applied onto one side of copper foil, dried and pressed, and then punched into 4 cm x 5 cm pieces to obtain an artificial graphite negative electrode for testing.

### (Production of Silicon-containing Graphite Negative Electrode)

To 85.0% by mass of artificial graphite powder, 7.0% by mass of nanosilicon, 3.0% by mass of conductive material (HS-100), 2.0% by mass of carbon nanofiber (VGCF), 2.0% by mass of styrene-butadiene rubber, 1.0% by mass of sodium carboxymethylcellulose, and water were mixed, and a negative electrode mixture paste was produced. The paste was applied onto one side of copper foil, and the resultant product was dried and pressed, and then punched into 4 cm × 5 cm pieces to obtain a silicon-containing graphite negative electrode for testing.

### (Production of Nonaqueous Electrolyte Solution Battery)

Under an argon atmosphere at a dew point of -50°C or lower, a terminal was welded to the above NCM622 positive electrode, and both sides of the welded product were then sandwiched between two polyethylene separators (5 cm × 6 cm), and the outside of the sandwiched product were placed between two artificial graphite negative electrodes to which a terminal had been welded in advance so that a surface of the negative electrode active material faced a surface of the positive electrode active material. The resultant product was put in an aluminum laminated bag having an opening on one side, the nonaqueous electrolyte solution was vacuum-injected into the bag, and the opening was then sealed with heat to produce aluminum laminated nonaqueous electrolyte solution batteries according to Examples and Comparative Examples. The nonaqueous electrolyte solutions described in Tables 1 to 8 were used.

In the nonaqueous electrolyte solution batteries according to Examples and Comparative Examples in Tables 9 to 16, NCM811 was used as the positive electrode and silicon-containing graphite was used as the negative electrode to produce the nonaqueous electrolyte solution batteries in the same way as in the nonaqueous electrolyte solution batteries according to Examples and Comparative Examples in Tables 1 to 8. The nonaqueous electrolyte solutions described in Tables 9 to 16 were used.

### [Evaluation]

### -Initial Charge and Discharge-

The produced nonaqueous electrolyte solution battery was put in a 25°C constant temperature bath and, in this state, connected to a charge and discharge device. Charge was performed at 3 mA until a voltage of 4.3 V was reached. After maintaining 4.3 V for 1 hour, the battery was discharged at 6 mA until a voltage of 2.7 V was reached. This was defined as one charge and discharge cycle, and three cycles of charge and discharge in total were performed to stabilize the battery.

### <Measurement of Initial Resistance>

After the initial charge and discharge, the battery was charged at 25°C and 6 mA until a voltage of 4.3 V was reached, and then the resistance value was measured by impedance measurement.

In each of Tables 1 to 16, the initial resistance value of Comparative Examples using the comparative nonaqueous electrolyte solution to which neither the component (I) nor a comparative compound had been added (Comparative Example 1-1 in Table 1, Comparative Example 2-1 in Table 2, Comparative Example 3-1 in Table 3, Comparative Example 4-1 in Table 4, Comparative Example 5-1 in Table 5, Comparative Example 6-1 in Table 6, Comparative Example 7-1 in Table 7, Comparative Example 8-1 in Table 8, Comparative Example 9-1 in Table 9, Comparative Example 10-1 in Table 10, Comparative Example 11-1 in Table 11, Comparative Example 12-1 in Table 12, Comparative Example 13-1 in Table 13, Comparative Example 14-1 in Table 14, Comparative Example 15-1 in Table 15, and Comparative Example 16-1 in Table 16) was defined as 100, and the evaluation results of the initial resistance in each of Examples and Comparative Examples were shown as relative values.

**Table 1**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 1-1 | Nonaqueous electrolyte solution 1-1 | (1-1-g) | 0.5 | - | - | 86.3 |
| Example 1-2 | Nonaqueous electrolyte solution 1-2 | (1-1-g) | 1.0 | - | - | 90.8 |
| Example 1-3 | Nonaqueous electrolyte solution 1-3 | (1-2-g) | 0.5 | - | - | 86.9 |
| Example 1-4 | Nonaqueous electrolyte solution 1-4 | (1-2-g) | 1.0 | - | - | 89.1 |
| Example 1-5 | Nonaqueous electrolyte solution 1-5 | (2-1-d) | 0.5 | - | - | 83.0 |
| Example 1-6 | Nonaqueous electrolyte solution 1-6 | (2-1-d) | 1.0 | - | - | 72.3 |
| Example 1-7 | Nonaqueous electrolyte solution 1-7 | (2-2-a) | 0.5 | - | - | 84.8 |
| Example 1-8 | Nonaqueous electrolyte solution 1-8 | (2-2-a) | 1.0 | - | - | 73.5 |
| Example 1-9 | Nonaqueous electrolyte solution 1-9 | (3-1-e) | 0.5 | - | - | 74.3 |
| Example 1-10 | Nonaqueous electrolyte solution 1-10 | (3-1-e) | 1.0 | - | - | 71.3 |
| Example 1-11 | Nonaqueous electrolyte solution 1-11 | (3-2-e) | 1.0 | - | - | 74.5 |
| Example 1-12 | Nonaqueous electrolyte solution 1-12 | (4-1-j) | 1.0 | - | - | 75.1 |
| Example 1-13 | Nonaqueous electrolyte solution 1-13 | (4-2-j) | 1.0 | - | - | 79.0 |
| Example 1-14 | Nonaqueous electrolyte solution 1-14 | (5-1-e) | 1.0 | - | - | 84.4 |
| Example 1-15 | Nonaqueous electrolyte solution 1-15 | (6-1-e) | 1.0 | - | - | 93.1 |
| Comparative Example 1-1 | Comparative nonaqueous electrolyte solution 1-1 | - | - | - | - | 100 |
| Comparative Example 1-2 | Comparative nonaqueous electrolyte solution 1-2 | MP | 0.5 | - | - | 101.5 |
| Comparative Example 1-3 | Comparative nonaqueous electrolyte solution 1-3 | MP | 1.0 | - | - | 103.6 |
| Comparative Example 1-4 | Comparative nonaqueous electrolyte solution 1-4 | DMI | 0.5 | - | - | 100.7 |
| Comparative Example 1-5 | Comparative nonaqueous electrolyte solution 1-5 | DMI | 1.0 | - | - | 103.1 |

**Table 2**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 2-1 | Nonaqueous electrolyte solution 2-1 | (1-1-g) | 1.0 | VC | 1.0 | 77.1 |
| Example 2-2 | Nonaqueous electrolyte solution 2-2 | (1-2-g) | 1.0 | VC | 1.0 | 78.1 |
| Example 2-3 | Nonaqueous electrolyte solution 2-3 | (2-1-d) | 1.0 | VC | 1.0 | 72.5 |
| Example 2-4 | Nonaqueous electrolyte solution 2-4 | (2-2-a) | 1.0 | VC | 1.0 | 73.6 |
| Example 2-5 | Nonaqueous electrolyte solution 2-5 | (3-1-e) | 1.0 | VC | 1.0 | 72.8 |
| Example 2-6 | Nonaqueous electrolyte solution 2-6 | (3-2-e) | 1.0 | VC | 1.0 | 75.8 |
| Example 2-7 | Nonaqueous electrolyte solution 2-7 | (4-1-j) | 1.0 | VC | 1.0 | 75.8 |
| Example 2-8 | Nonaqueous electrolyte solution 2-8 | (4-2-j) | 1.0 | VC | 1.0 | 74.1 |
| Example 2-9 | Nonaqueous electrolyte solution 2-9 | (5-1-e) | 1.0 | VC | 1.0 | 74.1 |
| Example 2-10 | Nonaqueous electrolyte solution 2-10 | (6-1-e) | 1.0 | VC | 1.0 | 85.3 |
| Comparative Example 2-1 | Comparative nonaqueous electrolyte solution 2-1 | - | - | VC | 1.0 | 100 |
| Comparative Example 2-2 | Comparative nonaqueous electrolyte solution 2-2 | MP | 1.0 | VC | 1.0 | 102.5 |
| Comparative Example 2-3 | Comparative nonaqueous electrolyte solution 2-3 | DMI | 1.0 | VC | 1.0 | 101.1 |

**Table 3**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 3-1 | Nonaqueous electrolyte solution 3-1 | (1-1-g) | 1.0 | BOB | 1.0 | 79.4 |
| Example 3-2 | Nonaqueous electrolyte solution 3-2 | (1-2-g) | 1.0 | BOB | 1.0 | 83.3 |
| Example 3-3 | Nonaqueous electrolyte solution 3-3 | (2-1-d) | 1.0 | BOB | 1.0 | 70.2 |
| Example 3-4 | Nonaqueous electrolyte solution 3-4 | (2-2-a) | 1.0 | BOB | 1.0 | 72.0 |
| Example 3-5 | Nonaqueous electrolyte solution 3-5 | (3-1-e) | 1.0 | BOB | 1.0 | 71.0 |
| Example 3-6 | Nonaqueous electrolyte solution 3-6 | (3-2-e) | 1.0 | BOB | 1.0 | 74.2 |
| Example 3-7 | Nonaqueous electrolyte solution 3-7 | (4-1-j) | 1.0 | BOB | 1.0 | 74.5 |
| Example 3-8 | Nonaqueous electrolyte solution 3-8 | (4-2-j) | 1.0 | BOB | 1.0 | 72.2 |
| Example 3-9 | Nonaqueous electrolyte solution 3-9 | (5-1-e) | 1.0 | BOB | 1.0 | 74.1 |
| Example 3-10 | Nonaqueous electrolyte solution 3-10 | (6-1-e) | 1.0 | BOB | 1.0 | 88.1 |
| Comparative Example 3-1 | Comparative nonaqueous electrolyte solution 3-1 | - | - | BOB | 1.0 | 100 |
| Comparative Example 3-2 | Comparative nonaqueous electrolyte solution 3-2 | MP | 1.0 | BOB | 1.0 | 100.1 |
| Comparative Example 3-3 | Comparative nonaqueous electrolyte solution 3-3 | DMI | 1.0 | BOB | 1.0 | 99.5 |

**Table 4**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 4-1 | Nonaqueous electrolyte solution 4-1 | (1-1-g) | 1.0 | DFBOP | 1.0 | 77.3 |
| Example 4-2 | Nonaqueous electrolyte solution 4-2 | (1-2-g) | 1.0 | DFBOP | 1.0 | 79.1 |
| Example 4-3 | Nonaqueous electrolyte solution 4-3 | (2-1-d) | 1.0 | DFBOP | 1.0 | 69.5 |
| Example 4-4 | Nonaqueous electrolyte solution 4-4 | (2-2-a) | 1.0 | DFBOP | 1.0 | 70.4 |
| Example 4-5 | Nonaqueous electrolyte solution 4-5 | (3-1-e) | 1.0 | DFBOP | 1.0 | 69.7 |
| Example 4-6 | Nonaqueous electrolyte solution 4-6 | (3-2-e) | 1.0 | DFBOP | 1.0 | 71.6 |
| Example 4-7 | Nonaqueous electrolyte solution 4-7 | (4-1-j) | 1.0 | DFBOP | 1.0 | 72.7 |
| Example 4-8 | Nonaqueous electrolyte solution 4-8 | (4-2-j) | 1.0 | DFBOP | 1.0 | 71.1 |
| Example 4-9 | Nonaqueous electrolyte solution 4-9 | (5-1-e) | 1.0 | DFBOP | 1.0 | 75.9 |
| Example 4-10 | Nonaqueous electrolyte solution 4-10 | (6-1-e) | 1.0 | DFBOP | 1.0 | 85.1 |
| Comparative Example 4-1 | Comparative nonaqueous electrolyte solution 4-1 | - | - | DFBOP | 1.0 | 100 |
| Comparative Example 4-2 | Comparative nonaqueous electrolyte solution 4-2 | MP | 1.0 | DFBOP | 1.0 | 101.2 |
| Comparative Example 4-3 | Comparative nonaqueous electrolyte solution 4-3 | DMI | 1.0 | DFBOP | 1.0 | 101.3 |

**Table 5**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 5-1 | Nonaqueous electrolyte solution 5-1 | (1-1-g) | 1.0 | TFOP | 1.0 | 73.2 |
| Example 5-2 | Nonaqueous electrolyte solution 5-2 | (1-2-g) | 1.0 | TFOP | 1.0 | 75.0 |
| Example 5-3 | Nonaqueous electrolyte solution 5-3 | (2-1-d) | 1.0 | TFOP | 1.0 | 68.1 |
| Example 5-4 | Nonaqueous electrolyte solution 5-4 | (2-2-a) | 1.0 | TFOP | 1.0 | 70.2 |
| Example 5-5 | Nonaqueous electrolyte solution 5-5 | (3-1-e) | 1.0 | TFOP | 1.0 | 69.2 |
| Example 5-6 | Nonaqueous electrolyte solution 5-6 | (3-2-e) | 1.0 | TFOP | 1.0 | 73.0 |
| Example 5-7 | Nonaqueous electrolyte solution 5-7 | (4-1-j) | 1.0 | TFOP | 1.0 | 73.1 |
| Example 5-8 | Nonaqueous electrolyte solution 5-8 | (4-2-j) | 1.0 | TFOP | 1.0 | 72.8 |
| Example 5-9 | Nonaqueous electrolyte solution 5-9 | (5-1-e) | 1.0 | TFOP | 1.0 | 71.0 |
| Example 5-10 | Nonaqueous electrolyte solution 5-10 | (6-1-e) | 1.0 | TFOP | 1.0 | 80.2 |
| Comparative Example 5-1 | Comparative nonaqueous electrolyte solution 5-1 | - | - | TFOP | 1.0 | 100 |
| Comparative Example 5-2 | Comparative nonaqueous electrolyte solution 5-2 | MP | 1.0 | TFOP | 1.0 | 100.2 |
| Comparative Example 5-3 | Comparative nonaqueous electrolyte solution 5-3 | DMI | 1.0 | TFOP | 1.0 | 102.2 |

**Table 6**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 6-1 | Nonaqueous electrolyte solution 6-1 | (1-1-g) | 1.0 | FSI | 1.0 | 79.5 |
| Example 6-2 | Nonaqueous electrolyte solution 6-2 | (1-2-g) | 1.0 | FSI | 1.0 | 78.6 |
| Example 6-3 | Nonaqueous electrolyte solution 6-3 | (2-1-d) | 1.0 | FSI | 1.0 | 69.1 |
| Example 6-4 | Nonaqueous electrolyte solution 6-4 | (2-2-a) | 1.0 | FSI | 1.0 | 71.1 |
| Example 6-5 | Nonaqueous electrolyte solution 6-5 | (3-1-e) | 1.0 | FSI | 1.0 | 71.0 |
| Example 6-6 | Nonaqueous electrolyte solution 6-6 | (3-2-e) | 1.0 | FSI | 1.0 | 74.2 |
| Example 6-7 | Nonaqueous electrolyte solution 6-7 | (4-1-j) | 1.0 | FSI | 1.0 | 72.2 |
| Example 6-8 | Nonaqueous electrolyte solution 6-8 | (4-2-j) | 1.0 | FSI | 1.0 | 74.1 |
| Example 6-9 | Nonaqueous electrolyte solution 6-9 | (5-1-e) | 1.0 | FSI | 1.0 | 77.2 |
| Example 6-10 | Nonaqueous electrolyte solution 6-10 | (6-1-e) | 1.0 | FSI | 1.0 | 85.4 |
| Comparative Example 6-1 | Comparative nonaqueous electrolyte solution 6-1 | - | - | FSI | 1.0 | 100 |
| Comparative Example 6-2 | Comparative nonaqueous electrolyte solution 6-2 | MP | 1.0 | FSI | 1.0 | 101.3 |
| Comparative Example 6-3 | Comparative nonaqueous electrolyte solution 6-3 | DMI | 1.0 | FSI | 1.0 | 100.0 |

**Table 7**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 7-1 | Nonaqueous electrolyte solution 7-1 | (1-1-g) | 1.0 | DFP | 1.0 | 84.2 |
| Example 7-2 | Nonaqueous electrolyte solution 7-2 | (1-2-g) | 1.0 | DFP | 1.0 | 79.7 |
| Example 7-3 | Nonaqueous electrolyte solution 7-3 | (2-1-d) | 1.0 | DFP | 1.0 | 66.1 |
| Example 7-4 | Nonaqueous electrolyte solution 7-4 | (2-2-a) | 1.0 | DFP | 1.0 | 69.4 |
| Example 7-5 | Nonaqueous electrolyte solution 7-5 | (3-1-e) | 1.0 | DFP | 1.0 | 68.2 |
| Example 7-6 | Nonaqueous electrolyte solution 7-6 | (3-2-e) | 1.0 | DFP | 1.0 | 70.2 |
| Example 7-7 | Nonaqueous electrolyte solution 7-7 | (4-1-j) | 1.0 | DFP | 1.0 | 73.4 |
| Example 7-8 | Nonaqueous electrolyte solution 7-8 | (4-2-j) | 1.0 | DFP | 1.0 | 72.1 |
| Example 7-9 | Nonaqueous electrolyte solution 7-9 | (5-1-e) | 1.0 | DFP | 1.0 | 77.3 |
| Example 7-10 | Nonaqueous electrolyte solution 7-10 | (6-1-e) | 1.0 | DFP | 1.0 | 85.2 |
| Comparative Example 7-1 | Comparative nonaqueous electrolyte solution 7-1 | - | - | DFP | 1.0 | 100 |
| Comparative Example 7-2 | Comparative nonaqueous electrolyte solution 7-2 | MP | 1.0 | DFP | 1.0 | 104.4 |
| Comparative Example 7-3 | Comparative nonaqueous electrolyte solution 7-3 | DMI | 1.0 | DFP | 1.0 | 102.8 |

**Table 8**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 8-1 | Nonaqueous electrolyte solution 8-1 | (1-1-g) | 1.0 | FS | 1.0 | 77.5 |
| Example 8-2 | Nonaqueous electrolyte solution 8-2 | (1-2-g) | 1.0 | FS | 1.0 | 78.1 |
| Example 8-3 | Nonaqueous electrolyte solution 8-3 | (2-1-d) | 1.0 | FS | 1.0 | 74.9 |
| Example 8-4 | Nonaqueous electrolyte solution 8-4 | (2-2-a) | 1.0 | FS | 1.0 | 76.2 |
| Example 8-5 | Nonaqueous electrolyte solution 8-5 | (3-1-e) | 1.0 | FS | 1.0 | 76.0 |
| Example 8-6 | Nonaqueous electrolyte solution 8-6 | (3-2-e) | 1.0 | FS | 1.0 | 75.1 |
| Example 8-7 | Nonaqueous electrolyte solution 8-7 | (4-1-j) | 1.0 | FS | 1.0 | 77.6 |
| Example 8-8 | Nonaqueous electrolyte solution 8-8 | (4-2-j) | 1.0 | FS | 1.0 | 77.3 |
| Example 8-9 | Nonaqueous electrolyte solution 8-9 | (5-1-e) | 1.0 | FS | 1.0 | 76.1 |
| Example 8-10 | Nonaqueous electrolyte solution 8-10 | (6-1-e) | 1.0 | FS | 1.0 | 86.3 |
| Comparative Example 8-1 | Comparative nonaqueous electrolyte solution 8-1 | - | - | FS | 1.0 | 100 |
| Comparative Example 8-2 | Comparative nonaqueous electrolyte solution 8-2 | MP | 1.0 | FS | 1.0 | 103.5 |
| Comparative Example 8-3 | Comparative nonaqueous electrolyte solution 8-3 | DMI | 1.0 | FS | 1.0 | 102.9 |

**Table 9**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 9-1 | Nonaqueous electrolyte solution 9-1 | (1-1-g) | 0.5 | - | - | 88.2 |
| Example 9-2 | Nonaqueous electrolyte solution 9-2 | (1-1-g) | 1.0 | - | - | 90.9 |
| Example 9-3 | Nonaqueous electrolyte solution 9-3 | (1-2-g) | 0.5 | - | - | 87.9 |
| Example 9-4 | Nonaqueous electrolyte solution 9-4 | (1-2-g) | 1.0 | - | - | 89.6 |
| Example 9-5 | Nonaqueous electrolyte solution 9-5 | (2-1-d) | 0.5 | - | - | 83.9 |
| Example 9-6 | Nonaqueous electrolyte solution 9-6 | (2-1-d) | 1.0 | - | - | 71.3 |
| Example 9-7 | Nonaqueous electrolyte solution 9-7 | (2-2-a) | 0.5 | - | - | 85.0 |
| Example 9-8 | Nonaqueous electrolyte solution 9-8 | (2-2-a) | 1.0 | - | - | 73.1 |
| Example 9-9 | Nonaqueous electrolyte solution 9-9 | (3-1-e) | 0.5 | - | - | 77.3 |
| Example 9-10 | Nonaqueous electrolyte solution 9-10 | (3-1-e) | 1.0 | - | - | 72.2 |
| Example 9-11 | Nonaqueous electrolyte solution 9-11 | (3-2-e) | 1.0 | - | - | 75.0 |
| Example 9-12 | Nonaqueous electrolyte solution 9-12 | (4-1-j) | 1.0 | - | - | 72.3 |
| Example 9-13 | Nonaqueous electrolyte solution 9-13 | (4-2-j) | 1.0 | - | - | 78.7 |
| Example 9-14 | Nonaqueous electrolyte solution 9-14 | (5-1-e) | 1.0 | - | - | 80.4 |
| Example 9-15 | Nonaqueous electrolyte solution 9-15 | (6-1-e) | 1.0 | - | - | 93.2 |
| Comparative Example 9-1 | Comparative nonaqueous electrolyte solution 9-1 | - | - | - | - | 100 |
| Comparative Example 9-2 | Comparative nonaqueous electrolyte solution 9-2 | MP | 0.5 | - | - | 102.3 |
| Comparative Example 9-3 | Comparative nonaqueous electrolyte solution 9-3 | MP | 1.0 | - | - | 103.7 |
| Comparative Example 9-4 | Comparative nonaqueous electrolyte solution 9-4 | DMI | 0.5 | - | - | 101.0 |
| Comparative Example 9-5 | Comparative nonaqueous electrolyte solution 9-5 | DMI | 1.0 | - | - | 102.7 |

**Table 10**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 10-1 | Nonaqueous electrolyte solution 10-1 | (1-1-g) | 1.0 | VC | 1.0 | 92.1 |
| Example 10-2 | Nonaqueous electrolyte solution 10-2 | (1-2-g) | 1.0 | VC | 1.0 | 84.5 |
| Example 10-3 | Nonaqueous electrolyte solution 10-3 | (2-1-d) | 1.0 | VC | 1.0 | 73.4 |
| Example 10-4 | Nonaqueous electrolyte solution 10-4 | (2-2-a) | 1.0 | VC | 1.0 | 75.5 |
| Example 10-5 | Nonaqueous electrolyte solution 10-5 | (3-1-e) | 1.0 | VC | 1.0 | 74.2 |
| Example 10-6 | Nonaqueous electrolyte solution 10-6 | (3-2-e) | 1.0 | VC | 1.0 | 77.8 |
| Example 10-7 | Nonaqueous electrolyte solution 10-7 | (4-1-j) | 1.0 | VC | 1.0 | 71.9 |
| Example 10-8 | Nonaqueous electrolyte solution 10-8 | (4-2-j) | 1.0 | VC | 1.0 | 72.8 |
| Example 10-9 | Nonaqueous electrolyte solution 10-9 | (5-1-e) | 1.0 | VC | 1.0 | 76.5 |
| Example 10-10 | Nonaqueous electrolyte solution 10-10 | (6-1-e) | 1.0 | VC | 1.0 | 87.2 |
| Comparative Example 10-1 | Comparative nonaqueous electrolyte solution 10-1 | - | - | VC | 1.0 | 100 |
| Comparative Example 10-2 | Comparative nonaqueous electrolyte solution 10-2 | MP | 1.0 | VC | 1.0 | 102.4 |
| Comparative Example 10-3 | Comparative nonaqueous electrolyte solution 10-3 | DMI | 1.0 | VC | 1.0 | 100.9 |

**Table 11**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 11-1 | Nonaqueous electrolyte solution 11-1 | (1-1-g) | 1.0 | BOB | 1.0 | 79.1 |
| Example 11-2 | Nonaqueous electrolyte solution 11-2 | (1-2-g) | 1.0 | BOB | 1.0 | 78.3 |
| Example 11-3 | Nonaqueous electrolyte solution 11-3 | (2-1-d) | 1.0 | BOB | 1.0 | 69.4 |
| Example 11-4 | Nonaqueous electrolyte solution 11-4 | (2-2-a) | 1.0 | BOB | 1.0 | 71.1 |
| Example 11-5 | Nonaqueous electrolyte solution 11-5 | (3-1-e) | 1.0 | BOB | 1.0 | 70.3 |
| Example 11-6 | Nonaqueous electrolyte solution 11-6 | (3-2-e) | 1.0 | BOB | 1.0 | 72.2 |
| Example 11-7 | Nonaqueous electrolyte solution 11-7 | (4-1-j) | 1.0 | BOB | 1.0 | 68.2 |
| Example 11-8 | Nonaqueous electrolyte solution 11-8 | (4-2-j) | 1.0 | BOB | 1.0 | 70.2 |
| Example 11-9 | Nonaqueous electrolyte solution 11-9 | (5-1-e) | 1.0 | BOB | 1.0 | 74.5 |
| Example 11-10 | Nonaqueous electrolyte solution 11-10 | (6-1-e) | 1.0 | BOB | 1.0 | 85.1 |
| Comparative Example 11-1 | Comparative nonaqueous electrolyte solution 11-1 | - | - | BOB | 1.0 | 100 |
| Comparative Example 11-2 | Comparative nonaqueous electrolyte solution 11-2 | MP | 1.0 | BOB | 1.0 | 100.5 |
| Comparative Example 11-3 | Comparative nonaqueous electrolyte solution 11-3 | DMI | 1.0 | BOB | 1.0 | 100.9 |

**Table 12**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 12-1 | Nonaqueous electrolyte solution 12-1 | (1-1-g) | 1.0 | DFBOP | 1.0 | 79.3 |
| Example 12-2 | Nonaqueous electrolyte solution 12-2 | (1-2-g) | 1.0 | DFBOP | 1.0 | 80.2 |
| Example 12-3 | Nonaqueous electrolyte solution 12-3 | (2-1-d) | 1.0 | DFBOP | 1.0 | 69.9 |
| Example 12-4 | Nonaqueous electrolyte solution 12-4 | (2-2-a) | 1.0 | DFBOP | 1.0 | 70.2 |
| Example 12-5 | Nonaqueous electrolyte solution 12-5 | (3-1-e) | 1.0 | DFBOP | 1.0 | 71.0 |
| Example 12-6 | Nonaqueous electrolyte solution 12-6 | (3-2-e) | 1.0 | DFBOP | 1.0 | 71.9 |
| Example 12-7 | Nonaqueous electrolyte solution 12-7 | (4-1-j) | 1.0 | DFBOP | 1.0 | 70.1 |
| Example 12-8 | Nonaqueous electrolyte solution 12-8 | (4-2-j) | 1.0 | DFBOP | 1.0 | 69.5 |
| Example 12-9 | Nonaqueous electrolyte solution 12-9 | (5-1-e) | 1.0 | DFBOP | 1.0 | 73.9 |
| Example 12-10 | Nonaqueous electrolyte solution 12-10 | (6-1-e) | 1.0 | DFBOP | 1.0 | 85.3 |
| Comparative Example 12-1 | Comparative nonaqueous electrolyte solution 12-1 | - | - | DFBOP | 1.0 | 100 |
| Comparative Example 12-2 | Comparative nonaqueous electrolyte solution 12-2 | MP | 1.0 | DFBOP | 1.0 | 103.5 |
| Comparative Example 12-3 | Comparative nonaqueous electrolyte solution 12-3 | DMI | 1.0 | DFBOP | 1.0 | 102.1 |

**Table 13**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 13-1 | Nonaqueous electrolyte solution 13-1 | (1-1-g) | 1.0 | TFOP | 1.0 | 81.8 |
| Example 13-2 | Nonaqueous electrolyte solution 13-2 | (1-2-g) | 1.0 | TFOP | 1.0 | 79.6 |
| Example 13-3 | Nonaqueous electrolyte solution 13-3 | (2-1-d) | 1.0 | TFOP | 1.0 | 68.9 |
| Example 13-4 | Nonaqueous electrolyte solution 13-4 | (2-2-a) | 1.0 | TFOP | 1.0 | 71.2 |
| Example 13-5 | Nonaqueous electrolyte solution 13-5 | (3-1-e) | 1.0 | TFOP | 1.0 | 69.9 |
| Example 13-6 | Nonaqueous electrolyte solution 13-6 | (3-2-e) | 1.0 | TFOP | 1.0 | 74.0 |
| Example 13-7 | Nonaqueous electrolyte solution 13-7 | (4-1-j) | 1.0 | TFOP | 1.0 | 70.1 |
| Example 13-8 | Nonaqueous electrolyte solution 13-8 | (4-2-j) | 1.0 | TFOP | 1.0 | 72.3 |
| Example 13-9 | Nonaqueous electrolyte solution 13-9 | (5-1-e) | 1.0 | TFOP | 1.0 | 72.1 |
| Example 13-10 | Nonaqueous electrolyte solution 13-10 | (6-1-e) | 1.0 | TFOP | 1.0 | 81.5 |
| Comparative Example 13-1 | Comparative nonaqueous electrolyte solution 13-1 | - | - | TFOP | 1.0 | 100 |
| Comparative Example 13-2 | Comparative nonaqueous electrolyte solution 13-2 | MP | 1.0 | TFOP | 1.0 | 102.1 |
| Comparative Example 13-3 | Comparative nonaqueous electrolyte solution 13-3 | DMI | 1.0 | TFOP | 1.0 | 105.2 |

**Table 14**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 14-1 | Nonaqueous electrolyte solution 14-1 | (1-1-g) | 1.0 | FSI | 1.0 | 78.3 |
| Example 14-2 | Nonaqueous electrolyte solution 14-2 | (1-2-g) | 1.0 | FSI | 1.0 | 76.9 |
| Example 14-3 | Nonaqueous electrolyte solution 14-3 | (2-1-d) | 1.0 | FSI | 1.0 | 69.0 |
| Example 14-4 | Nonaqueous electrolyte solution 14-4 | (2-2-a) | 1.0 | FSI | 1.0 | 73.1 |
| Example 14-5 | Nonaqueous electrolyte solution 14-5 | (3-1-e) | 1.0 | FSI | 1.0 | 71.4 |
| Example 14-6 | Nonaqueous electrolyte solution 14-6 | (3-2-e) | 1.0 | FSI | 1.0 | 73.2 |
| Example 14-7 | Nonaqueous electrolyte solution 14-7 | (4-1-j) | 1.0 | FSI | 1.0 | 72.2 |
| Example 14-8 | Nonaqueous electrolyte solution 14-8 | (4-2-j) | 1.0 | FSI | 1.0 | 73.1 |
| Example 14-9 | Nonaqueous electrolyte solution 14-9 | (5-1-e) | 1.0 | FSI | 1.0 | 70.2 |
| Example 14-10 | Nonaqueous electrolyte solution 14-10 | (6-1-e) | 1.0 | FSI | 1.0 | 80.4 |
| Comparative Example 14-1 | Comparative nonaqueous electrolyte solution 14-1 | - | - | FSI | 1.0 | 100 |
| Comparative Example 14-2 | Comparative nonaqueous electrolyte solution 14-2 | MP | 1.0 | FSI | 1.0 | 101.5 |
| Comparative Example 14-3 | Comparative nonaqueous electrolyte solution 14-3 | DMI | 1.0 | FSI | 1.0 | 103.2 |

**Table 15**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 15-1 | Nonaqueous electrolyte solution 15-1 | (1-1-g) | 1.0 | DFP | 1.0 | 75.9 |
| Example 15-2 | Nonaqueous electrolyte solution 15-2 | (1-2-g) | 1.0 | DFP | 1.0 | 77.4 |
| Example 15-3 | Nonaqueous electrolyte solution 15-3 | (2-1-d) | 1.0 | DFP | 1.0 | 68.1 |
| Example 15-4 | Nonaqueous electrolyte solution 15-4 | (2-2-a) | 1.0 | DFP | 1.0 | 69.0 |
| Example 15-5 | Nonaqueous electrolyte solution 15-5 | (3-1-e) | 1.0 | DFP | 1.0 | 65.2 |
| Example 15-6 | Nonaqueous electrolyte solution 15-6 | (3-2-e) | 1.0 | DFP | 1.0 | 70.9 |
| Example 15-7 | Nonaqueous electrolyte solution 15-7 | (4-1-j) | 1.0 | DFP | 1.0 | 69.4 |
| Example 15-8 | Nonaqueous electrolyte solution 15-8 | (4-2-j) | 1.0 | DFP | 1.0 | 72.4 |
| Example 15-9 | Nonaqueous electrolyte solution 15-9 | (5-1-e) | 1.0 | DFP | 1.0 | 73.3 |
| Example 15-10 | Nonaqueous electrolyte solution 15-10 | (6-1-e) | 1.0 | DFP | 1.0 | 82.2 |
| Comparative Example 15-1 | Comparative nonaqueous electrolyte solution 15-1 | - | - | DFP | 1.0 | 100 |
| Comparative Example 15-2 | Comparative nonaqueous electrolyte solution 15-2 | MP | 1.0 | DFP | 1.0 | 105.2 |
| Comparative Example 15-3 | Comparative nonaqueous electrolyte solution 15-3 | DMI | 1.0 | DFP | 1.0 | 104.1 |

**Table 16**

| | Nonaqueous electrolyte solution | Component (I) or comparative compound | | Other additive | | Initial resistance (relative value) |
|---|---|---|---|---|---|---|
| | | Type | Content [% by mass] | Type | Content [% by mass] | |
| Example 16-1 | Nonaqueous electrolyte solution 16-1 | (1-1-g) | 1.0 | FS | 1.0 | 83.5 |
| Example 16-2 | Nonaqueous electrolyte solution 16-2 | (1-2-g) | 1.0 | FS | 1.0 | 81.3 |
| Example 16-3 | Nonaqueous electrolyte solution 16-3 | (2-1-d) | 1.0 | FS | 1.0 | 72.4 |
| Example 16-4 | Nonaqueous electrolyte solution 16-4 | (2-2-a) | 1.0 | FS | 1.0 | 75.1 |
| Example 16-5 | Nonaqueous electrolyte solution 16-5 | (3-1-e) | 1.0 | FS | 1.0 | 76.0 |
| Example 16-6 | Nonaqueous electrolyte solution 16-6 | (3-2-e) | 1.0 | FS | 1.0 | 75.2 |
| Examples 16-7 | Nonaqueous electrolyte solution 16-7 | (4-1-j) | 1.0 | FS | 1.0 | 73.6 |
| Examples 16-8 | Nonaqueous electrolyte solution 16-8 | (4-2-j) | 1.0 | FS | 1.0 | 74.2 |
| Examples 16-9 | Nonaqueous electrolyte solution 16-9 | (5-1-e) | 1.0 | FS | 1.0 | 76.4 |
| Example 16-10 | Nonaqueous electrolyte solution 16-10 | (6-1-e) | 1.0 | FS | 1.0 | 83.1 |
| Comparative Example 16-1 | Comparative nonaqueous electrolyte solution 16-1 | - | - | FS | 1.0 | 100 |
| Comparative Example 16-2 | Comparative nonaqueous electrolyte solution 16-2 | MP | 1.0 | FS | 1.0 | 104.0 |
| Comparative Example 16-3 | Comparative nonaqueous electrolyte solution 16-3 | DMI | 1.0 | FS | 1.0 | 102.1 |

As is clear from Tables 1 to 16, it is found that a nonaqueous electrolyte solution battery using a nonaqueous electrolyte solution including the component (I) of the present disclosure has low initial resistance and is therefore excellent.

### INDUSTRIAL APPLICABILITY

According to the present disclosure, a nonaqueous electrolyte solution and a nonaqueous electrolyte solution battery which can reduce an initial resistance value can be provided. The present disclosure also allows for providing a compound which can be suitably used in the above nonaqueous electrolyte solution.

Although the present disclosure has been described in detail and with reference to specific examples, it will be apparent to those skilled in the art that various changes and modifications can be made without departing from the spirit and scope of the present disclosure.

The present application is based on a Japanese Patent Application (No. 2022-110786) filed on July 8, 2022 and a Japanese Patent Application (No. 2022-165369) filed on October 14, 2022, the contents of which are incorporated herein by reference.

## Claims

1. A nonaqueous electrolyte solution comprising:
(I) at least one selected from the group consisting of a compound represented by the following Formula (1), a compound represented by the following Formula (2), a compound represented by the following Formula (3), a compound represented by the following Formula (4-1), a compound represented by the following Formula (4-2), a compound represented by the following Formula (4-3), a compound represented by the following Formula (5), and a compound represented by the following Formula (6);
(II) a solute; and
(III) a nonaqueous organic solvent, 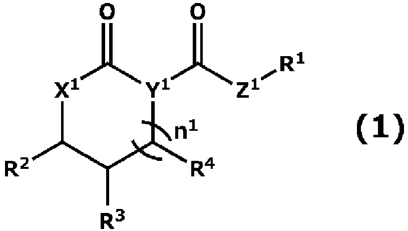
wherein in Formula (1), X¹ represents CH₂, NH, O, S, or SO₂,
Y¹ represents CH or N,
Z¹ represents CH₂, O, or NR⁵,
R⁵ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R⁵ represents an alkali metal cation, a bond between a nitrogen atom and R³ represents an ionic bond,
R¹ represents PO(R_{f})₂ or SO₂R_{f},
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of Rᵣ may be the same or different,
n¹ represents an integer of 1 to 3, and
R², R³, and R⁴ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when there are a plurality of R⁴, the plurality of R⁴ may be the same or different, 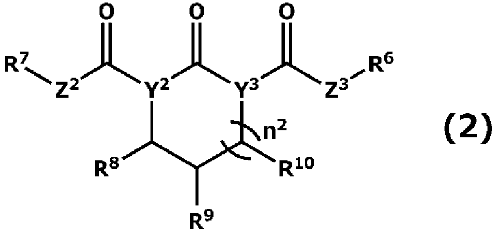
wherein in Formula (2), Y² and Y³ each independently represent CH or N,
Z² and Z³ each independently represent CH₂, O, or NR¹¹,
R¹¹ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R¹¹ represents an alkali metal cation, a bond between a nitrogen atom and R¹¹ represents an ionic bond,
R⁶ and R⁷ each independently represent PO(R_{f})₂ or SO₂R_{f},
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of R_{f} may be the same or different,
n² represents an integer of 1 to 3, and
R⁸, R⁹, and R¹⁰ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when there are a plurality of R¹⁰, the plurality of R¹⁰ may be the same or different, 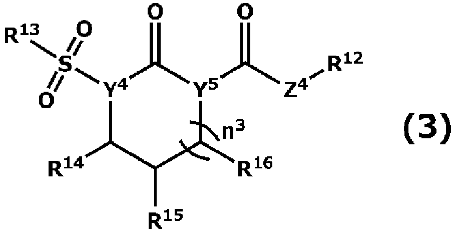
wherein in Formula (3), Y⁴ and Y^{s} each independently represent CH or N,
Z⁴ represents CH₂, O, or NR¹⁷,
R¹⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R¹⁷ represents an alkali metal cation, a bond between a nitrogen atom and R¹⁷ represents an ionic bond,
R¹² represents PO(R_{f})₂ or SO₂R_{f},
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of R_{f} may be the same or different,
R¹³ represents a halogen atom, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom,
n³ represents an integer of 0 to 3,
R¹⁴, R¹⁵, and R¹⁶ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; when there are a plurality of R¹⁶, the plurality of R¹⁶ may be the same or different; and when n³ represents 0, there is no R¹⁶, and a carbon atom to which R¹⁵ is bonded is directly bonded to Y⁵, 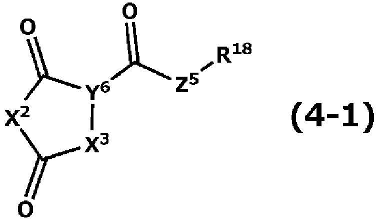
wherein in Formula (4-1), X² and X³ each independently represent CH₂, NH, O, or S,
Y⁶ represents CH or N,
Z⁵ represents CH₂, O, or NR^{z3},
R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²³ represents an alkali metal cation, a bond between a nitrogen atom and R²³ represents an ionic bond,
R¹⁸ represents PO(R_{f})₂ or SO₂R_{f}, and
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of R_{f} may be the same or different, 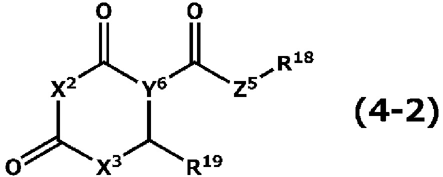
wherein in Formula (4-2), X² and X³ each independently represent CH₂, NH, O, or S,
Y⁶ represents CH or N,
Z⁵ represents CH₂, O, or NR²³,
R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²³ represents an alkali metal cation, a bond between a nitrogen atom and R²³ represents an ionic bond,
R¹⁸ represents PO(R_{f})₂ or SO₂R_{f},
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of R_{f} may be the same or different, and
R¹⁹ represents a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom, 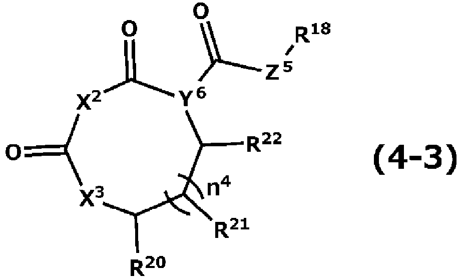
wherein in Formula (4-3), X² and X³ each independently represent CH₂, NH, O, or S,
Y⁶ represents CH or N,
Z⁵ represents CH₂, O, or NR²³,
R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²³ represents an alkali metal cation, a bond between a nitrogen atom and R²³ represents an ionic bond,
R¹⁸ represents PO(R_{f})₂ or SO₂R_{f},
Rᵣ represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of R_{f} may be the same or different,
n⁴ represents 0 or 1, and
R²⁰, R²¹, and R²² each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when n⁴ represents 0, there is no R²¹, and a carbon atom to which R²⁰ is bonded is directly bonded to a carbon atom to which R²² is bonded, 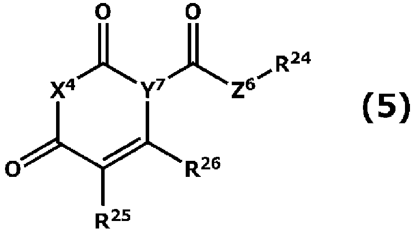
wherein in Formula (5), X⁴ represents CH₂, NH, O, or S,
Y' represents CH or N,
Z⁶ represents CH₂, O, or NR²',
R²⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²⁷ represents an alkali metal cation, a bond between a nitrogen atom and R²⁷ represents an ionic bond,
R²⁴ represents PO(R_{f})₂ or SO₂R_{f},
R_{f} represents a halogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; any hydrogen atom of the alkyl group, the alkenyl group, or the alkynyl group may be substituted with a fluorine atom; and when there are a plurality of R_{f}, the plurality of R_{f} may be the same or different, and
R²⁵ and R²⁶ each independently represent a halogen atom, a hydrogen atom, a nitro group, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom, and 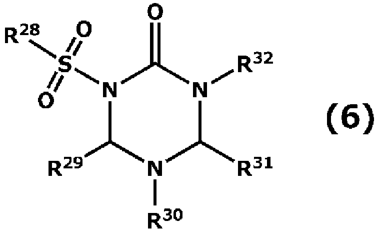
wherein in Formula (6), R²⁸ represents a halogen atom, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom,
R²⁹ and R³¹ each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom, and
R³⁰ and R³² each independently represent a hydrogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; a carbon-nitrogen unsaturated bond may be included in the alkyl group, the alkenyl group, or the alkynyl group; and any hydrogen atom of the alkyl group, the alkenyl group, or the alkynyl group may be substituted with a fluorine atom.

2. The nonaqueous electrolyte solution according to claim 1, wherein
the above-described (I) is at least one selected from the group consisting of the compound represented by Formula (2), the compound represented by Formula (3), the compound represented by Formula (4-1), the compound represented by Formula (4-2), and the compound represented by Formula (5).

3. The nonaqueous electrolyte solution according to claim 1, wherein
R^{I} in Formula (1) represents POF₂ or SO₂F.

4. The nonaqueous electrolyte solution according to claim 1 or 2, wherein
R⁶ and R⁷ in Formula (2) each independently represent POF₂ or SO₂F.

5. The nonaqueous electrolyte solution according to claim 1 or 2, wherein
R¹² in Formula (3) represents POF₂ or SO₂F.

6. The nonaqueous electrolyte solution according to claim 1 or 2, wherein
R¹⁸ in Formula (4-1), (4-2), or (4-3) represents POF₂ or SO₂F.

7. The nonaqueous electrolyte solution according to claim 1 or 2, wherein
R²⁴ in Formula (5) represents POF₂ or SO₂F.

8. The nonaqueous electrolyte solution according to claim 1, wherein
R²⁸ in Formula (6) represents a fluorine atom.

9. The nonaqueous electrolyte solution according to claim 1 or 3, wherein
R⁵ in Formula (1) represents a hydrogen atom, a lithium ion, a sodium ion, a linear alkyl group having 1 to 4 carbon atoms, or a branched alkyl group having 3 to 4 carbon atoms.

10. The nonaqueous electrolyte solution according to claim 1 or 2, wherein
R¹¹ in Formula (2) represents a hydrogen atom, a lithium ion, a sodium ion, a linear alkyl group having 1 to 4 carbon atoms, or a branched alkyl group having 3 to 4 carbon atoms.

11. The nonaqueous electrolyte solution according to claim 1 or 2, wherein
R¹⁷ in Formula (3) represents a hydrogen atom, a lithium ion, a sodium ion, a linear alkyl group having 1 to 4 carbon atoms, or a branched alkyl group having 3 to 4 carbon atoms.

12. The nonaqueous electrolyte solution according to claim 1 or 2, wherein
R²³ in Formula (4-1), (4-2), or (4-3) represents a hydrogen atom, a lithium ion, a sodium ion, a linear alkyl group having 1 to 4 carbon atoms, or a branched alkyl group having 3 to 4 carbon atoms.

13. The nonaqueous electrolyte solution according to claim 1 or 2, wherein
R²⁷ in Formula (5) represents a hydrogen atom, a lithium ion, a sodium ion, a linear alkyl group having 1 to 4 carbon atoms, or a branched alkyl group having 3 to 4 carbon atoms.

14. The nonaqueous electrolyte solution according to claim 1 or 2, wherein
the solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and Lil, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

15. The nonaqueous electrolyte solution according to claim 1 or 2, wherein
the nonaqueous organic solvent comprises at least one selected from the group consisting of a cyclic ester, a chain ester, a cyclic ether, a chain ether, a sulfone compound, a sulfoxide compound, and an ionic liquid.

16. The nonaqueous electrolyte solution according to claim 15, wherein
the cyclic ester is a cyclic carbonate.

17. The nonaqueous electrolyte solution according to claim 16, wherein
the cyclic carbonate is at least one selected from the group consisting of ethylene carbonate, propylene carbonate, and fluoroethylene carbonate.

18. The nonaqueous electrolyte solution according to claim 15, wherein
the chain ester is a chain carbonate.

19. The nonaqueous electrolyte solution according to claim 18, wherein
the chain carbonate is at least one selected from the group consisting of ethyl methyl carbonate, dimethyl carbonate, diethyl carbonate, and methyl propyl carbonate.

20. The nonaqueous electrolyte solution according to claim 1 or 2, wherein
a content of the above-described (I) is 0.01% to 10.0% by mass with respect to a total amount of the nonaqueous electrolyte solution.

21. The nonaqueous electrolyte solution according to claim 1 or 2, further comprising:
at least one selected from the group consisting of vinylene carbonate, bis(oxalato)borate, difluorooxalatoborate, difluorobis(oxalato)phosphate, tetrafluorooxalatophosphate, tetrafluoro(malonato)phosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(picolinato)phosphate, 1,3-dimethyl-1,3-divinyl-1,3-di(1,1,1,3,3,3-hexafluoroisopropyl)disiloxane, t-butylbenzene, t-amylbenzene, fluorobenzene, and cyclohexylbenzene.

22. A nonaqueous electrolyte solution battery at least comprising:
a positive electrode;
a negative electrode;
a separator; and
the nonaqueous electrolyte solution according to claim 1 or 2.

23. A compound represented by the following Formula (1), 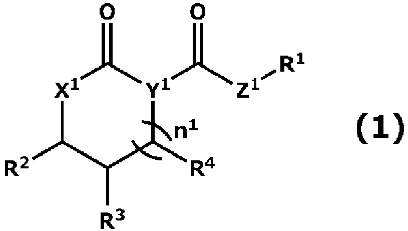
wherein in Formula (1), X' represents CH₂, NH, O, S, or SOz,
Y¹ represents CH or N,
Z¹ represents CH₂, O, or NR⁵
R⁵ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R⁵ represents an alkali metal cation, a bond between a nitrogen atom and R⁵ represents an ionic bond,
R¹ represents POF₃ or SO₂F,
n¹ represents an integer of 1 to 3, and
R², R³, and R⁴ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when there are a plurality of R⁴, the plurality of R⁴ may be the same or different.

24. A compound represented by the following Formula (2), 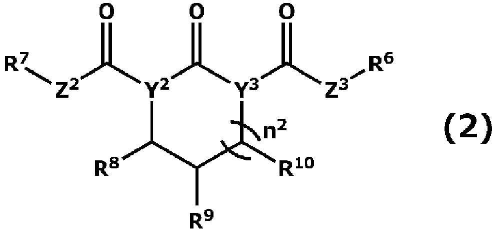
wherein in Formula (2), Y² and Y³ each independently represent CH or N,
Z² and Z³ each independently represent CH₂, O, σr NR¹¹,
R¹¹ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R¹¹ represents an alkali metal cation, a bond between a nitrogen atom and R¹¹ represents an ionic bond,
R⁶ and R⁷ each independently represent POF₂ or SO₂F,
n² represents an integer of 1 to 3, and
R⁸, R⁹, and R¹⁰ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when there are a plurality of R¹⁰, the plurality of R¹⁰ may be the same or different.

25. A compound represented by the following Formula (3), 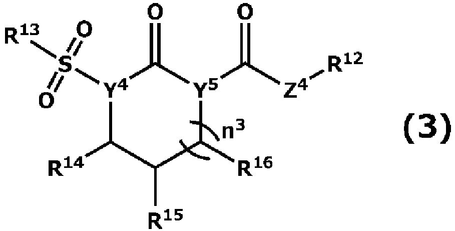
wherein in Formula (3), Y⁴ and ^{y5} each independently represent CH or N,
Z⁴ represents CH₂, O, or NR¹⁷,
R¹⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R¹⁷ represents an alkali metal cation, a bond between a nitrogen atom and R¹⁷ represents an ionic bond,
R¹² represents POF₂ σ_{F} ^{S02F,}
R¹³ represents a halogen atom, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom, and
n³ represents an integer of 0 to 3,
R¹⁴, R¹⁵, and R¹⁶ each independently represent a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; when there are a plurality of R¹⁶, the plurality of R¹⁶ may be the same or different, and when n³ represents 0, there is no R¹⁶, and a carbon atom to which R¹⁵ is bonded is directly bonded to Y⁵

26. A compound represented by the following Formula (4-1), the following Formula (4-2), or the following Formula (4-3), 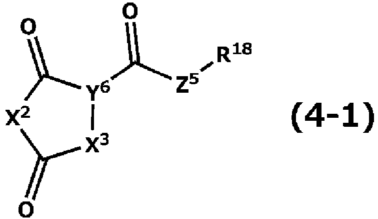
wherein in Formula (4-1), X² and X³ each independently represent CH₂, NH, O, or S,
Y⁶ represents CH or N,
Z⁵ represents CH₂, O, or NR²³,
R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²³ represents an alkali metal cation, a bond between a nitrogen atom and R²³ represents an ionic bond, and
R¹⁸ represents POF₂ or SO₂F, 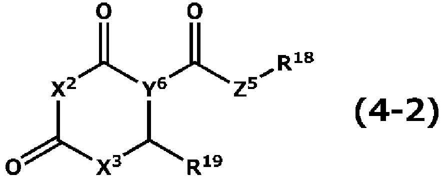
wherein in Formula (4-2), X² and X³ each independently represent CH₂, NH, O, or S,
Y⁶ represents CH or N,
Z⁵ represents CH₂, O, or NR²³,
R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²³ represents an alkali metal cation, a bond between a nitrogen atom and R²³ represents an ionic bond,
R¹⁸ represents POF₂ or SO₂F, and
R¹⁹ represents a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom, and 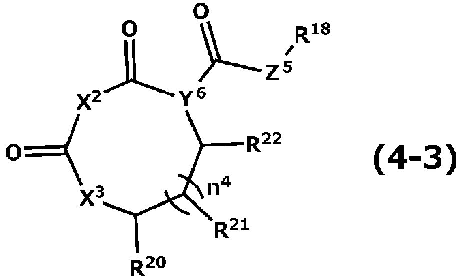
wherein in Formula (4-3), X² and X³ each independently represent CH₂, NH, O, or S,
Y⁶ represents CH or N,
Z⁵ represents CH₂, O, or NR²³,
R²³ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²³ represents an alkali metal cation, a bond between a nitrogen atom and R²³ represents an ionic bond,
R¹⁸ represents POF₂ or SO₂F,
n⁴ represents 0 or 1, and
R²⁰, R²¹, and R²² each independently represent a halogen atom, a hydrogen atom, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when n⁴ represents 0, there is no R²¹, and a carbon atom to which R²⁰ is bonded is directly bonded to a carbon atom to which R²² is bonded.

27. A compound represented by the following Formula (5), 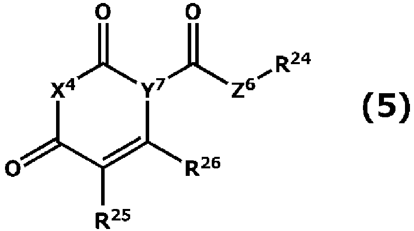
wherein in Formula (5), X⁴ represents CH₂, NH, or O,
Y¹ represents CH or N,
Z⁶ represents CH₂, O, or R²⁷
R²⁷ represents a hydrogen atom, an alkali metal cation, a linear alkyl group having 1 to 12 carbon atoms, or a branched alkyl group having 3 to 12 carbon atoms, and an oxygen atom may be included between a carbon atom-carbon atom bond in the alkyl group; any hydrogen atom of the alkyl group may be substituted with a fluorine atom; and when R²⁷ represents an alkali metal cation, a bond between a nitrogen atom and R²⁷ represents an ionic bond,
R²⁴ represents POF₂ or SO₂F, and
R²⁵ and R²⁶ each independently represent a halogen atom, a hydrogen atom, a nitro group, a linear alkyl group having 1 to 12 carbon atoms, or a branched alkyl group having 3 to 12 carbon atoms, and an oxygen atom may be included between a carbon atom-carbon atom bond in the alkyl group; and any hydrogen atom of the alkyl group may be substituted with a fluorine atom.

28. A compound represented by the following Formula (6), 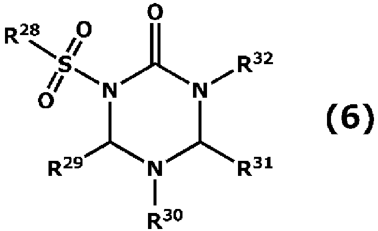
wherein in Formula (6), R²⁸ represents a fluorine atom,
R²⁹ and R³¹ each independently represent a halogen atom, a hydrogen atom, a linear alkyl group having 1 to 12 carbon atoms, or a branched alkyl group having 3 to 12 carbon atoms, an oxygen atom may be included between a carbon atom-carbon atom bond in the alkyl group; and any hydrogen atom of the alkyl group may be substituted with a fluorine atom, and
R³⁰ and R³² each independently represent a hydrogen atom, a linear alkyl group having 1 to 12 carbon atoms, a branched alkyl group having 3 to 12 carbon atoms, a linear alkenyl group having 2 to 12 carbon atoms, a branched alkenyl group having 3 to 12 carbon atoms, a linear alkynyl group having 2 to 12 carbon atoms, or a branched alkynyl group having 4 to 12 carbon atoms, and at least one of an oxygen atom and a nitrogen atom may be included between a carbon atom-carbon atom bond in the alkyl group, the alkenyl group, or the alkynyl group; a carbon-nitrogen unsaturated bond may be included in the alkyl group, the alkenyl group, or the alkynyl group; and any hydrogen atom of the alkyl group, the alkenyl group, or the alkynyl group may be substituted with a fluorine atom.
